# EUROPEAN PATENT APPLICATION

(11) **EP 3 498 187 A1**
(43) Date of publication of application: **19.06.2019**
(21) Application number: 18212708.4
(22) Date of filing: 14.12.2018
(51) Int. Cl.: A61B 17/072

(54) **SYSTEMS FOR CONTROLLING A CLAMPING MEMBER**

(30) Priority: 15.12.2017 US 201715843689
(71) Applicant: Ethicon LLC, Guaynabo, PR 00969 (US)
(72) Inventor: SHELTON, IV, Frederick E., Cincinnati, Ohio 45242 (US); WORTHINGTON, Sarah A., Cincinnati, Ohio 45242 (US); HARRIS, Jason L., Cincinnati, Ohio 45242 (US)
(74) Representative: Carpmaels & Ransford LLP

(57) **Abstract**

Various systems and methods of controlling a surgical instrument are disclosed. The surgical instrument includes a motor, a current sensor configured to sense a current drawn by the motor, and a control circuit coupled to the motor and the current sensor. The motor can be coupled to a clamping member, which is configured to transition an end effector between an open position and a closed position, cut tissue, and/or eject staples from a staple cartridge in the end effector. The control circuit can be configured to detect whether the current drawn by the motor exceeds a threshold via the current sensor and, upon detecting that the current drawn by the motor exceeds the threshold, control the motor to change a speed at which the clamping member is driven.

## Description

### BACKGROUND

The present invention relates to surgical instruments and, in various arrangements, to surgical stapling and cutting instruments and staple cartridges for use therewith that are designed to staple and cut tissue.

While several devices have been made and used, it is believed that no one prior to the inventors has made or used the device described in the appended claims.

### SUMMARY

In one aspect, a surgical instrument comprising: a motor; a current sensor configured to sense a current drawn by the motor; and a control circuit coupled to the motor and the current sensor, the control circuit configured to: detect a position of a clamping member drivable by the motor between a first position and a second position, wherein the clamping member is configured to: transition an end effector to a closed position as the clamping member moves from the first position to the second position; and deploy a plurality of staples from a cartridge positioned in the end effector after the end effector is in the closed position as the clamping member moves to the second position; wherein the control circuit is further configured to: detect whether the current drawn by the motor exceeds a threshold via the current sensor; and upon detecting that the current drawn by the motor exceeds the threshold, control the motor to change a speed at which the clamping member is driven.

In another aspect, a surgical instrument comprising: a motor; a current sensor configured to sense a current drawn by the motor; and a control circuit coupled to the motor and the current sensor, the control circuit configured to: detect a position of a clamping member drivable by the motor between a first position and a second position, wherein the clamping member is configured to: transition an end effector to a closed position as the clamping member moves from the first position to the second position; and deploy a plurality of staples from a cartridge positioned in the end effector after the end effector is in the closed position as the clamping member moves to the second position; wherein the control circuit is further configured to: control the motor to change a speed at which the clamping member is driven at a defined position between the first position and the second position; and detect the defined position according to the current drawn by the motor via the current sensor.

### FIGURES

Various features of the embodiments described herein, together with advantages thereof, may be understood in accordance with the following description taken in conjunction with the accompanying drawings as follows:
FIG. 1 is a perspective view of an electromechanical surgical system;
FIG. 2 is a perspective view of a distal end of an electromechanical surgical instrument portion of the surgical system of FIG. 1;
FIG. 3 is an exploded assembly view of an outer shell feature and the electromechanical surgical instrument of FIG. 2;
FIG. 4 is a rear perspective view of a portion of the electromechanical surgical instrument of FIG. 2;
FIG. 5 is a partial exploded assembly view of a portion of an adapter and the electromechanical surgical instrument of the surgical system of FIG. 1;
FIG. 6 is an exploded assembly view of a portion of the adapter of FIG. 5;
FIG. 7 is a cross-sectional perspective view of a portion of an articulation assembly of an adapter;
FIG. 8 is a perspective view of the articulation assembly of FIG. 7;
FIG. 9 is another perspective view of the articulation assembly of FIG. 8;
FIG. 10 is an exploded assembly view of a loading unit employed in the electromechanical surgical system of FIG. 1;
FIG. 11 is a perspective view of an alternative adapter embodiment;
FIG. 12 is a side elevational view of a portion of a loading unit of the adapter of FIG. 11 with the jaws thereof in an open position;
FIG. 13 is another side elevational view of a portion of the loading unit of FIG. 11 with portions thereof shown in cross-section and the jaws thereof in a closed position;
FIG. 14 is a bottom view of a portion of the loading unit of FIG. 13 with portions thereof shown in cross-section;
FIG. 15 is a perspective view of a portion of the loading unit of FIG. 15 with a portion of the outer tube shown in phantom lines;
FIG. 16 is a schematic diagram of a circuit for controlling a motor of a surgical instrument;
FIG. 17 is a schematic diagram of a circuit for controlling a motor of a surgical instrument;
FIG. 18 is a schematic diagram of a position sensor of a surgical instrument;
FIG. 19 is a logic flow diagram of a process for controlling a speed of a clamping member during a firing stroke;
FIG. 20 is a logic flow diagram of a process for detecting a defined position according to motor current;
FIG. 21 is a graph of various clamping member firing strokes executed per the logic depicted in FIGS. 19 and 20;
FIG. 22 is an exploded view of an anvil including a slot stop member;
FIG. 23 is a partial cutaway view of an anvil including a slot stop member;
FIG. 24 is a sectional view of an anvil including a slot stop member;
FIG. 25 is a side elevational view of an anvil including a slot stop member;
FIG. 26 is a longitudinal sectional view of an end effector and a drive assembly including a stop member, with the clamping member in a proximal position;
FIG. 27 is a longitudinal sectional view of an end effector and a drive assembly including a stop member, with the clamping member in a distal position;
FIG. 28 is a longitudinal sectional view of an end effector including a stop member located distally in the elongated slot, with the clamping member in a proximal position;
FIG. 29 is a longitudinal sectional view of an end effector including a stop member located distally in the elongated slot, with the clamping member in a distal position; and
FIG. 30 is a cross-sectional view of the adapter.

### DESCRIPTION

Applicant of the present application owns the following U.S. Patent Applications that were filed on even date herewith and which are each herein incorporated by reference in their respective entireties:
- U.S. Patent Application Serial No. __________, entitled SEALED ADAPTERS FOR USE WITH ELECTROMECHANICAL SURGICAL INSTRUMENTS; Attorney Docket No. END8286USNP/170227;
- U.S. Patent Application Serial No. __________, entitled END EFFECTORS WITH POSITIVE JAW OPENING FEATURES FOR USE WITH ADAPTERS FOR ELECTROMECHANICAL SURGICAL INSTRUMENTS; Attorney Docket No. END8277USNP/170219;
- U.S. Patent Application Serial No. __________, entitled SURGICAL END EFFECTORS WITH CLAMPING ASSEMBLIES CONFIGURED TO INCREASE JAW APERTURE RANGES; Attorney Docket No. END8278USNP/170220;
- U.S. Patent Application Serial No. __________, entitled SURGICAL END EFFECTORS WITH PIVOTAL JAWS CONFIGURED TO TOUCH AT THEIR RESPECTIVE DISTAL ENDS WHEN FULLY CLOSED; Attorney Docket No. END8283USNP/170223;
- U.S. Patent Application Serial No. __________, entitled SURGICAL END EFFECTORS WITH JAW STIFFENER ARRANGEMENTS CONFIGURED TO PERMIT MONITORING OF FIRING MEMBER; Attorney Docket No. END8282USNP/170221;
- U.S. Patent Application Serial No. __________, entitled ADAPTERS WITH END EFFECTOR POSITION SENSING AND CONTROL ARRANGEMENTS FOR USE IN CONNECTION WITH ELECTROMECHANICAL SURGICAL INSTRUMENTS; Attorney Docket No. END8281USNP/170228;
- U.S. Patent Application Serial No. __________, entitled DYNAMIC CLAMPING ASSEMBLIES WITH IMPROVED WEAR CHARACTERISTICS FOR USE IN CONNECTION WITH ELECTROMECHANICAL SURGICAL INSTRUMENTS; Attorney Docket No. END8279USNP/170222;
- U.S. Patent Application Serial No. __________, entitled ADAPTERS WITH FIRING STROKE SENSING ARRANGEMENTS FOR USE IN CONNECTION WITH ELECTROMECHANICAL SURGICAL INSTRUMENTS; Attorney Docket No. END8287USNP/170229;
- U.S. Patent Application Serial No. __________, entitled ADAPTERS WITH CONTROL SYSTEMS FOR CONTROLLING MULTIPLE MOTORS OF AN ELECTRICAL MECHANICAL SURGICAL INSTRUMENT; Attorney Docket No. END8284USNP/170224;
- U.S. Patent Application Serial No. __________, entitled HANDHELD ELECTROMECHANICAL SURGICAL INSTRUMENTS WITH IMPROVED MOTOR CONTROL ARRANGEMENTS FOR POSITIONING COMPONENTS OF AN ADAPTER COUPLED THERETO; Attorney Docket No. END8285USNP/170255;
- U.S. Patent Application Serial No. __________, entitled SYSTEMS AND METHODS OF CONTROLLING A CLAMPING MEMBER FIRING RATE OF A SURGICAL INSTRUMENT; Attorney Docket No. END8280USNP/170226; and
- U.S. Patent Application Serial No. __________, entitled METHODS OF OPERATING SURGICAL END EFFECTORS; Attorney Docket No. END8298USNP/170218M.

Numerous specific details are set forth to provide a thorough understanding of the overall structure, function, manufacture, and use of the embodiments as described in the specification and illustrated in the accompanying drawings. Well-known operations, components, and elements have not been described in detail so as not to obscure the embodiments described in the specification. The reader will understand that the embodiments described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications including, for example, in connection with open surgical procedures. As the present Detailed Description proceeds, the reader will further appreciate that the various instruments disclosed herein can be inserted into a body in anyway, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other embodiments are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other embodiments are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other embodiments are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

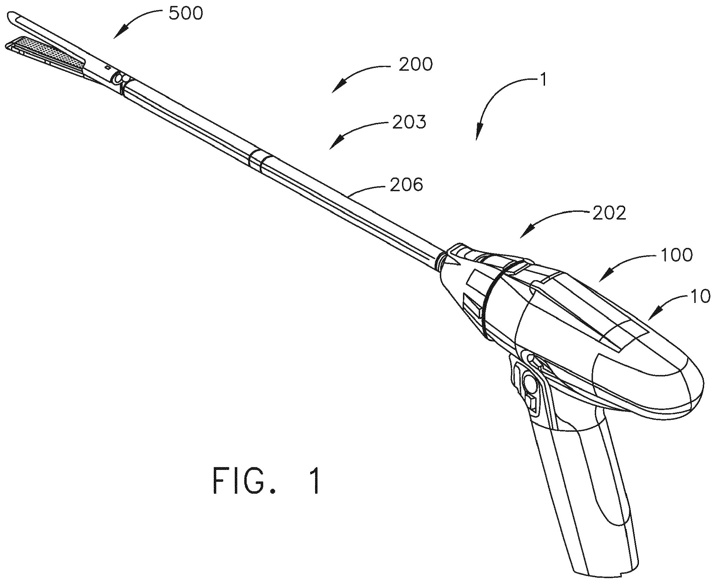

FIG. 1 depicts a motor-driven (electromechanical) surgical system 1 that may be used to perform a variety of different surgical procedures. As can be seen in that Figure, one example of the surgical system 1 includes a powered handheld electromechanical surgical instrument 100 that is configured for selective attachment thereto of a plurality of different surgical tool implements (referred to herein as "adapters") that are each configured for actuation and manipulation by the powered handheld electromechanical surgical instrument. As illustrated in FIG. 1, the handheld surgical instrument 100 is configured for selective connection with an adapter 200, and, in turn, adapter 200 is configured for selective connection with end effectors that comprise a single use loading unit ("SULU") or a disposable loading unit ("DLU") or a multiple use loading unit ("MULU"). In another surgical system embodiment, various forms of adapter 200 may also be effectively employed with a tool drive assembly of a robotically controlled or automated surgical system. For example, the surgical tool assemblies disclosed herein may be employed with various robotic systems, instruments, components and methods such as, but not limited to, those disclosed in U.S. Patent No. 9,072,535, entitled SURGICAL STAPLING INSTRUMENTS WITH ROTATABLE STAPLE DEPLOYMENT ARRANGEMENTS, which is hereby incorporated by reference herein in its entirety.

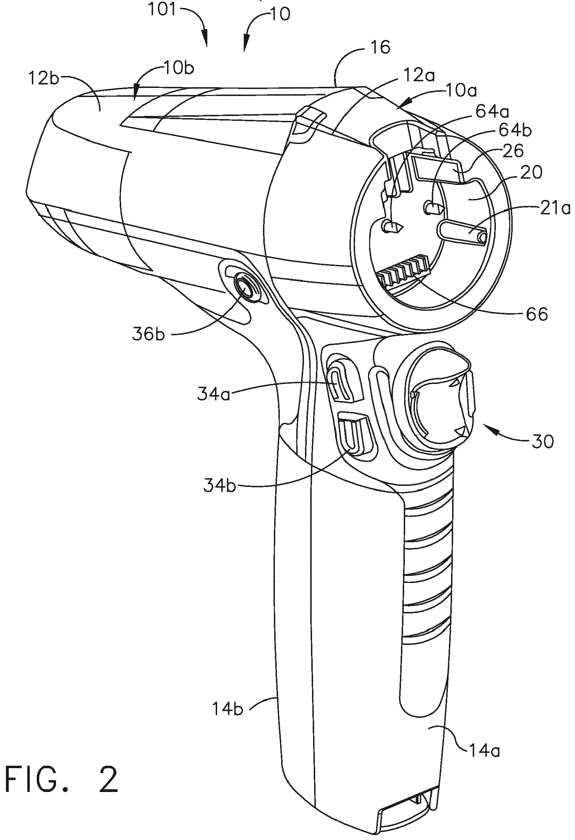

As illustrated in FIGS. 1 and 2, surgical instrument 100 includes a power-pack 101 and an outer shell housing 10 that is configured to selectively receive and substantially encase the power-pack 101. The power pack 101 may also be referred to herein as handle assembly 101. One form of surgical instrument 100, for example, is disclosed in International Publication No. WO 2016/057225 A1, International Application No. PCT/US2015/051837, entitled HANDHELD ELECTROMECHANICAL SURGICAL SYSTEM, the entire disclosure of which is hereby incorporated by reference herein. Various features of surgical instrument 100 will not be disclosed herein beyond what is necessary to understand the various features of the inventions disclosed herein with it being understood that further details may be gleaned from reference to WO 2016/057225 and other references incorporated by reference herein.

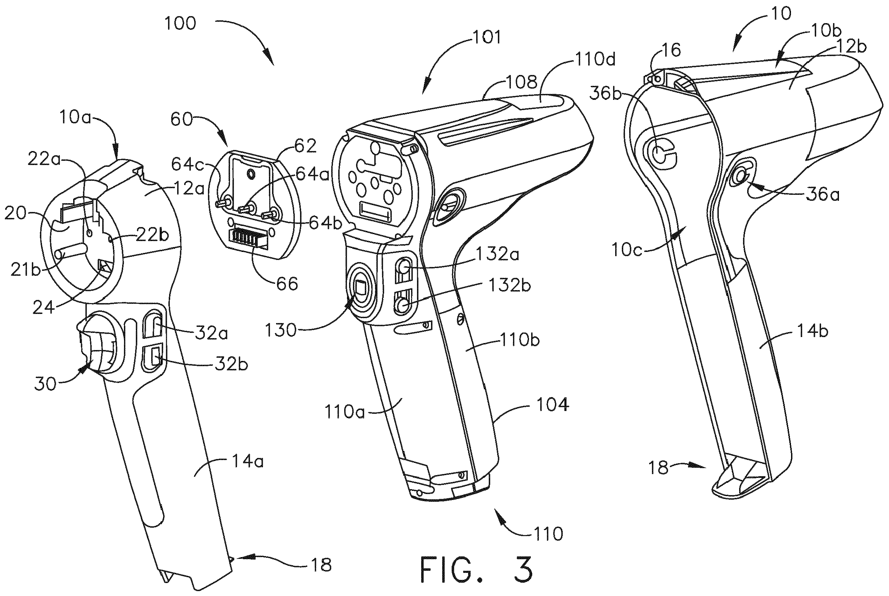

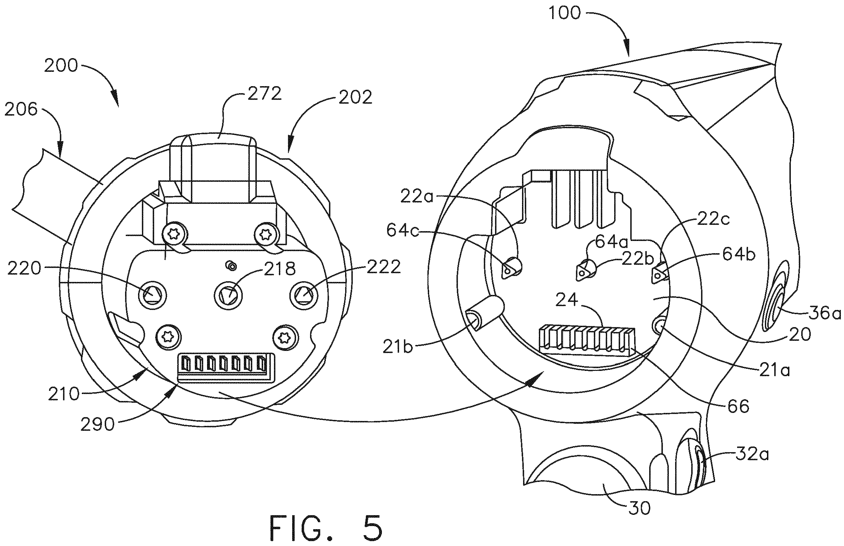

As illustrated in FIG. 3, outer shell housing 10 includes a distal half-section 10a and a proximal half-section 10b that is pivotably connected to distal half-section 10a by a hinge 16 located along an upper edge of distal half-section 10a and proximal half-section 10b. When joined, distal and proximal half-sections 10a, 10b define a shell cavity 10c therein in which the power-pack 101 is selectively situated. Each of distal and proximal half-sections 10a, 10b includes a respective upper shell portion 12a, 12b, and a respective lower shell portion 14a, 14b. Lower shell portions 14a, 14b define a snap closure feature 18 for selectively securing the lower shell portions 14a, 14b to one another and for maintaining shell housing 10 in a closed condition. Distal half-section 10a of shell housing 10 defines a connecting portion 20 that is configured to accept a corresponding drive coupling assembly 210 of adapter 200 (see FIG. 5). Specifically, distal half-section 10a of shell housing 10 has a recess that receives a portion of drive coupling assembly 210 of adapter 200 when adapter 200 is mated to surgical instrument 100.

Connecting portion 20 of distal half-section 10a defines a pair of axially extending guide rails 21a, 21b that project radially inward from inner side surfaces thereof as shown in FIG. 5. Guide rails 21a, 21b assist in rotationally orienting adapter 200 relative to surgical instrument 100 when adapter 200 is mated to surgical instrument 100. Connecting portion 20 of distal half-section 10a defines three apertures 22a, 22b, 22c that are formed in a distally facing surface thereof and which are arranged in a common plane or line with one another. Connecting portion 20 of distal half-section 10a also defines an elongate slot 24 also formed in the distally facing surface thereof. Connecting portion 20 of distal half-section 10a further defines a female connecting feature 26 (see FIG. 2) formed in a surface thereof. Female connecting feature 26 selectively engages with a male connecting feature of adapter 200.

Distal half-section 10a of shell housing 10 supports a distal facing toggle control button 30. The toggle control button 30 is capable of being actuated in a left, right, up and down direction upon application of a corresponding force thereto or a depressive force thereto. Distal half-section 10a of shell housing 10 supports a right-side pair of control buttons 32a, 32b (see FIG. 3); and a left-side pair of control button 34a, 34b (see FIG. 2). The right-side control buttons 32a, 32b and the left-side control buttons 34a, 34b are capable of being actuated upon application of a corresponding force thereto or a depressive force thereto. Proximal half-section 10b of shell housing 10 supports a right-side control button 36a (see Fig 3) and a left-side control button 36b (see FIG. 2). Right-side control button 36a and left-side control button36b are capable of being actuated upon application of a corresponding force thereto or a depressive force thereto.

Shell housing 10 includes a sterile barrier plate assembly 60 selectively supported in distal half-section 10a. Specifically, the sterile barrier plate assembly 60 is disposed behind connecting portion 20 of distal half-section 10a and within shell cavity 10c of shell housing 10. The plate assembly 60 includes a plate 62 rotatably supporting three coupling shafts 64a, 64b, 64c (see FIGS. 3 and 5). Each coupling shaft 64a, 64b, 64c extends from opposed sides of plate 62 and has a tri-lobe transverse cross-sectional profile. Each coupling shaft 64a, 64b, 64c extends through the respective apertures 22a, 22b, 22c of connecting portion 20 of distal half-section 10a when the sterile barrier plate assembly 60 is disposed within shell cavity 10c of shell housing 10. The plate assembly 60 further includes an electrical pass-through connector 66 supported on plate 62. Pass-through connector 66 extends from opposed sides of plate 62. Pass-through connector 66 defines a plurality of contact paths each including an electrical conduit for extending an electrical connection across plate 62. When the plate assembly 60 is disposed within shell cavity 10c of shell housing 10, distal ends of coupling shaft 64a, 64b, 64c and a distal end of pass-through connector 66 are disposed or situated within connecting portion 20 of distal half-section 10a of shell housing 10, and are configured to electrically and/or mechanically engage respective corresponding features of adapter 200.

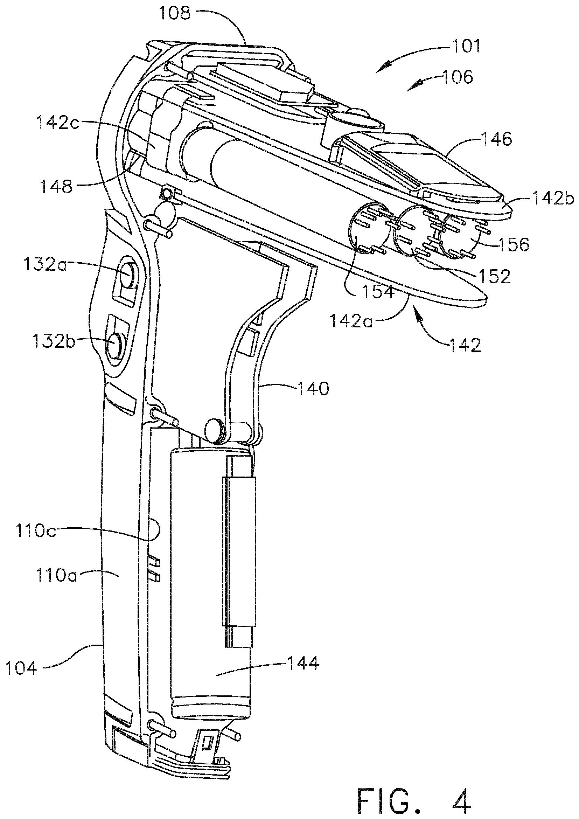

Referring to FIGS. 3 and 4, the power-pack or the handle assembly 101 includes an inner handle housing 110 having a lower housing portion 104 and an upper housing portion 108 extending from and/or supported on lower housing portion 104. Lower housing portion 104 and upper housing portion 108 are separated into a distal half section 110a and a proximal half-section 110b connectable to distal half-section 110a by a plurality of fasteners. When joined, distal and proximal half-sections 110a, 110b define the inner handle housing 110 having an inner housing cavity 110c therein in which a power-pack core assembly 106 is situated. Power- pack core assembly 106 is configured to control the various operations of surgical instrument 100.

Distal half-section 110a of inner handle housing 110 supports a distal toggle control interface 130 that is in operative registration with the distal toggle control button 30 of shell housing 10. In use, when the power-pack 101 is disposed within shell housing 10, actuation of the toggle control button 30 exerts a force on toggle control interface 130. Distal half-section 110a of inner handle housing 110 also supports a right-side pair of control interfaces (not shown), and a left-side pair of control interfaces 132a, 132b. In use, when the power-pack 101 is disposed within shell housing 10, actuation of one of the right-side pair of control buttons or the left-side pair of control button of distal half-section 10a of shell housing 10 exerts a force on a respective one of the right-side pair of control interfaces 132a, 132b or the left-side pair of control interfaces 132a, 132b of distal half-section 110a of inner handle housing 110.

With reference to FIGS. 1-5, inner handle housing 110 provides a housing in which power-pack core assembly 106 is situated. Power-pack core assembly 106 includes a battery circuit 140, a controller circuit board 142 and a rechargeable battery 144 configured to supply power to any of the electrical components of surgical instrument 100. Controller circuit board 142 includes a motor controller circuit board 142a, a main controller circuit board 142b, and a first ribbon cable 142c interconnecting motor controller circuit board 142a and main controller circuit board 142b. Power-pack core assembly 106 further includes a display screen 146 supported on main controller circuit board 142b. Display screen 146 is visible through a clear or transparent window 110d (see FIG. 3) provided in proximal half-section 110b of inner handle housing 110. It is contemplated that at least a portion of inner handle housing 110 may be fabricated from a transparent rigid plastic or the like. It is further contemplated that shell housing 10 may either include a window formed therein (in visual registration with display screen 146 and with window 110d of proximal half-section 110b of inner handle housing 110, and/or shell housing 10 may be fabricated from a transparent rigid plastic or the like.

Power-pack core assembly 106 further includes a first motor 152, a second motor 154, and a third motor 156 that are supported by motor bracket 148 and are each electrically connected to controller circuit board 142 and battery 144. Motors 152, 154, 156 are disposed between motor controller circuit board 142a and main controller circuit board 142b. Each motor 152, 154, 156 includes a respective motor shaft 152a, 154a, 156a extending therefrom. Each motor shaft 152a, 154a, 156a has a tri-lobe transverse cross-sectional profile for transmitting rotative forces or torque. Each motor 152, 154, 156 is controlled by a respective motor controller. Rotation of motor shafts 152a, 154a, 156a by respective motors 152, 154, 156 function to drive shafts and/or gear components of adapter 200 in order to perform the various operations of surgical instrument 100. In particular, motors 152, 154, 156 of power-pack core assembly 106 are configured to drive shafts and/or gear components of adapter 200.

As illustrated in FIGS. 1 and 5, surgical instrument 100 is configured for selective connection with adapter 200, and, in turn, adapter 200 is configured for selective connection with end effector 500. Adapter 200 includes an outer knob housing 202 and an outer tube 206 that extends from a distal end of knob housing 202. Knob housing 202 and outer tube 206 are configured and dimensioned to house the components of adapter assembly 200. Outer tube 206 is dimensioned for endoscopic insertion, in particular, that outer tube is passable through a typical trocar port, cannula or the like. Knob housing 202 is dimensioned to not enter the trocar port, cannula of the like. Knob housing 202 is configured and adapted to connect to connecting portion 20 of the outer shell housing 10 of surgical instrument 100.

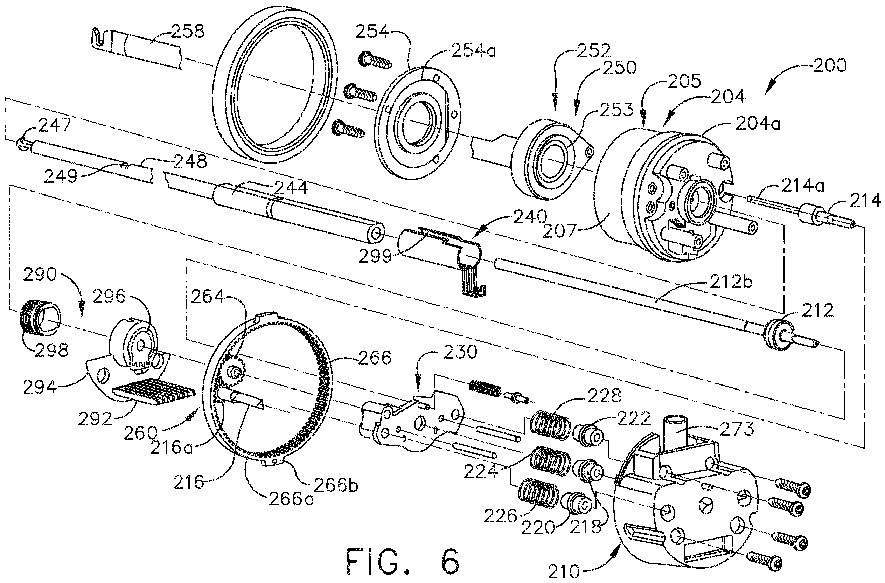

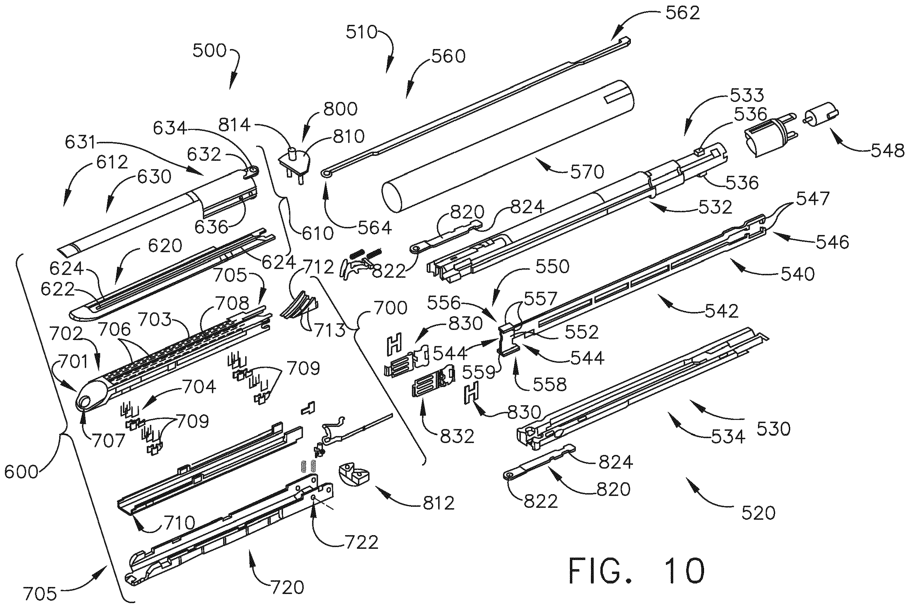

Adapter 200 is configured to convert a rotation of either of first or second coupling shafts 64a, 64b of surgical instrument 100 into axial translation useful for operating a drive assembly 540 and an articulation link 560 of end effector 500, as illustrated in FIG. 10 and as will be described in greater detail below. As illustrated in FIG. 6, adapter 200 includes the proximal inner housing assembly 204 that rotatably supports a first rotatable proximal drive shaft 212, a second rotatable proximal drive shaft 214, and a third rotatable proximal drive shaft 216 therein. Each proximal drive shaft 212, 214, 216 functions as a rotation receiving member to receive rotational forces from respective coupling shafts 64a, 64b and 64c of surgical instrument 100. In addition, the drive coupling assembly 210 of adapter 200 is also configured to rotatably support first, second and third connector sleeves 218, 220 and 222, respectively, arranged in a common plane or line with one another. Each connector sleeve 218, 220, 222 is configured to mate with respective first, second and third coupling shafts 64a, 64b, 64c of surgical instrument 100, as described above. Each connector sleeves 218, 222, 220 is further configured to mate with a proximal end of respective first, second, and third proximal drive shafts 212, 214, 216 of adapter 200.

Drive coupling assembly 210 of adapter 200 also includes a first, a second, and a third biasing member 224, 226, and 228 disposed distally of respective first, second, and third connector sleeves 218, 220, 222. Each biasing members 224, 226, and 228 is disposed about respective first, second, and third rotatable proximal drive shaft 212, 214, and 216. Biasing members 224, 226, and 228 act on respective connector sleeves 218, 222, and 220 to help maintain connector sleeves 218, 222. and 220 engaged with the distal end of respective coupling shafts 64a, 64b, and 64c of surgical instrument 100 when adapter 200 is connected to surgical instrument 100.

Also in the illustrated arrangement, adapter 200 includes first, second, and third drive converting assemblies 240, 250, 260, respectively, that are each disposed within inner housing assembly 204 and outer tube 206. Each drive converting assembly 240, 250, 260 is configured and adapted to transmit or convert a rotation of a first, second, and third coupling shafts 64a, 64b, and 64c of surgical instrument 100 into axial translation of an articulation driver or bar 258 of adapter 200, to effectuate articulation of end effector 500; a rotation of a ring gear 266 of adapter 200, to effectuate rotation of adapter 200; or axial translation of a distal drive member 248 of adapter 200 to effectuate closing, opening, and firing of end effector 500.

Still referring to FIG. 6, first force/rotation transmitting/converting assembly 240 includes first rotatable proximal drive shaft 212, which, as described above, is rotatably supported within inner housing assembly 204. First rotatable proximal drive shaft 212 includes a non-circular or shaped proximal end portion configured for connection with first connector sleeve 218 which is connected to respective first coupling shaft 64a of surgical instrument 100. First rotatable proximal drive shaft 212 includes a threaded distal end portion 212b. First force/rotation transmitting/converting assembly 240 further includes a drive coupling nut 244 that threadably engages the threaded distal end portion 212b of first rotatable proximal drive shaft 212, and which is slidably disposed within outer tube 206. Drive coupling nut 244 is slidably keyed within proximal core tube portion of outer tube 206 so as to be prevented from rotation as first rotatable proximal drive shaft 212 is rotated. In this manner, as the first rotatable proximal drive shaft 212 is rotated, drive coupling nut 244 is translated along threaded distal end portion 212b of first rotatable proximal drive shaft 212 and, in turn, through and/or along outer tube 206.

First force/rotation transmitting/converting assembly 240 further includes a distal drive member 248 that is mechanically engaged with drive coupling nut 244, such that axial movement of drive coupling nut 244 results in a corresponding amount of axial movement of distal drive member 248. The distal end portion of distal drive member 248 supports a connection member 247 configured and dimensioned for selective engagement with an engagement member 546 of a drive assembly 540 of end effector 500 (FIG. 10). Drive coupling nut 244 and/or distal drive member 248 function as a force transmitting member to components of end effector 500. In operation, as first rotatable proximal drive shaft 212 is rotated, as a
result of the rotation of first coupling shaft 64a of surgical instrument 100, drive coupling nut 244 is translated axially along first rotatable proximal drive shaft 212. As drive coupling nut 244 is translated axially along first rotatable proximal drive shaft 212, distal drive member 248 is translated axially relative to outer tube 206. As distal drive member 248 is translated axially, with connection member 247 connected thereto and engaged with a hollow drive member 548 attached to drive assembly 540 of end effector 500 (FIG. 10), distal drive member 248 causes concomitant axial translation of drive assembly 540 of end effector 500 to effectuate a closure of a tool assembly portion 600 of the end effector 500 and a firing of various components within the tool assembly.

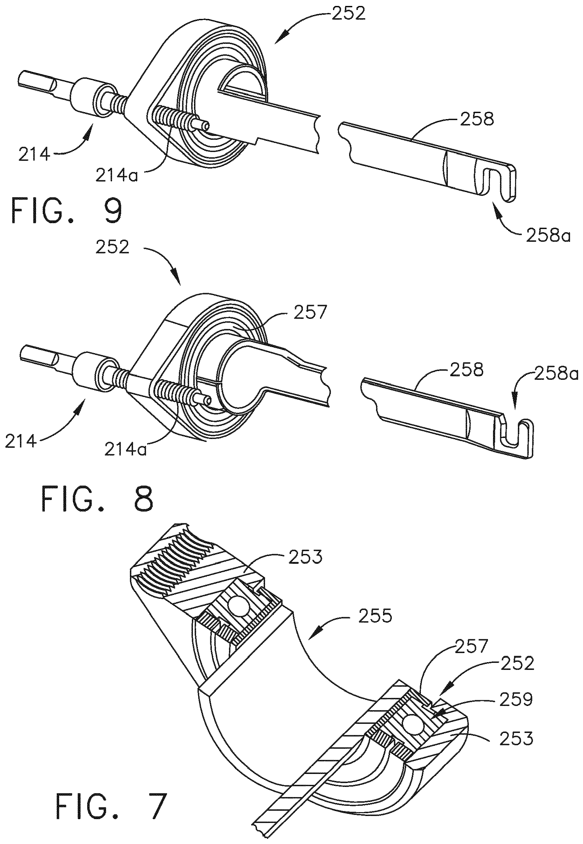

Still referring to FIG. 6, second drive converting assembly 250 of adapter 200 includes second proximal drive shaft 214 that is rotatably supported within inner housing assembly 204. Second rotatable proximal drive shaft 214 includes a non-circular or shaped proximal end portion configured for connection with second coupling shaft 64c of surgical instrument 100. Second rotatable proximal drive shaft 214 further includes a threaded distal end portion 214a configured to threadably engage an articulation bearing housing 253 of an articulation bearing assembly 252. Referring to FIGS. 6-9, the articulation bearing housing 253 supports an articulation bearing 255 that has an inner race 257 that is independently rotatable relative to an outer race 259. Articulation bearing housing 253 has a non-circular outer profile, for example tear-dropped shaped, that is slidably and non-rotatably disposed within a complementary bore (not shown) of inner housing hub 204a. Second drive converting assembly 250 of adapter 200 further includes articulation bar 258 that has a proximal portion that is secured to inner race 257 of articulation bearing 255. A distal portion of articulation bar 258 includes a slot 258a therein, which is configured to accept a hook 562 the articulation link 560 (FIG. 10) of end effector 500. Articulation bar 258 functions as a force transmitting member to components of end effector 500. In the illustrated arrangement and as further discussed in WO 2016/057225 A1, articulation bearing assembly 252 is both rotatable and longitudinally translatable and is configured to permit free, unimpeded rotational movement of end effector 500 when its first and second jaw members 610, 700 are in an approximated position and/or when jaw members 610, 700 are articulated.

In operation, as second proximal drive shaft 214 is rotated, the articulation bearing assembly 252 is axially translated along threaded distal end portion 214a of second proximal drive shaft 214, which in turn, causes articulation bar 258 to be axially translated relative to outer tube 206. As articulation bar 258 is translated axially, articulation bar 258, being coupled to articulation link 560 of end effector 500, causes concomitant axial translation of articulation link 560 of end effector 500 to effectuate an articulation of tool assembly 600. Articulation bar 258 is secured to inner race 257 of articulation bearing 253 and is thus free to rotate about the longitudinal axis relative to outer race 259 of articulation bearing 253.

As illustrated in FIG. 6, adapter 200 includes a third drive converting assembly 260 that is supported in inner housing assembly 204. Third drive converting assembly 260 includes rotation ring gear 266 that is fixedly supported in and connected to outer knob housing 202. Ring gear 266 defines an internal array of gear teeth 266a and includes a pair of diametrically opposed, radially extending protrusions 266b. Protrusions 266b are configured to be disposed within recesses defined in outer knob housing 202, such that rotation of ring gear 266 results in rotation of outer knob housing 202, and vice a versa. Third drive converting assembly 260 further includes third rotatable proximal drive shaft 216 which, as described above, is rotatably supported within inner housing assembly 204. Third rotatable proximal drive shaft 216 includes a non-circular or shaped proximal end portion that is configured for connection with third connector 220. Third rotatable proximal drive shaft 216 includes a spur gear 216 keyed to a distal end thereof. A reversing spur gear 264 inter-engages spur gear 216a of third rotatable proximal drive shaft 216 to gear teeth 266a of ring gear 266. In operation, as third rotatable proximal drive shaft 216 is rotated, due to a rotation of the third coupling shaft 64b of surgical instrument 100, spur gear 216a of third rotatable proximal drive shaft 216 engages reversing gear 264 causing reversing gear 264 to rotate. As reversing gear 264 rotates, ring gear 266 also rotates thereby causing outer knob housing 202 to rotate. Rotation of the outer knob housing 202 causes the outer tube 206 to rotate about longitudinal axis of adapter 200. As outer tube 206 is rotated, end effector 500 that is connected to a distal end portion of adapter 200, is also rotated about a longitudinal axis of adapter 200.

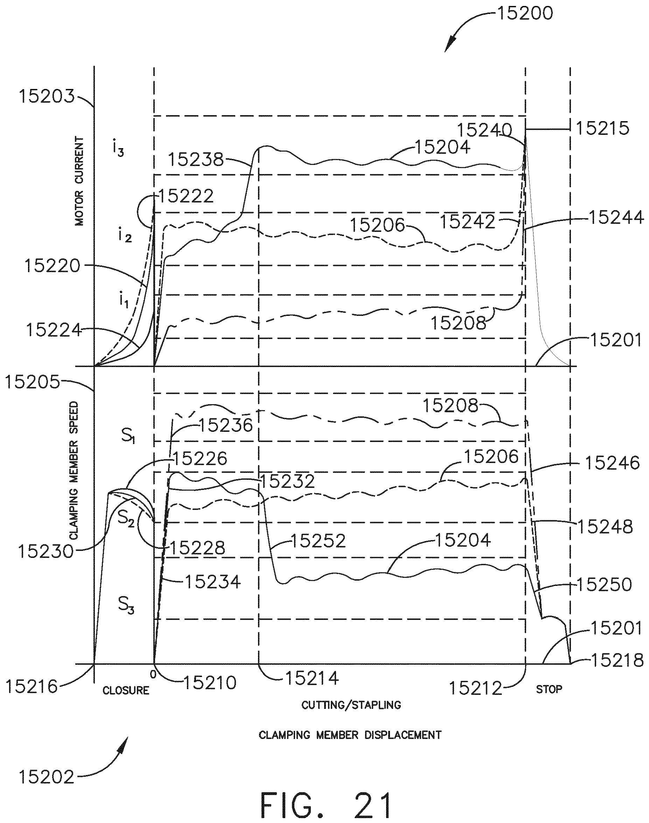

Adapter 200 further includes an attachment/detachment button 272 (FIG. 5) that is supported on a stem 273 (FIG. 6) that projects from drive coupling assembly 210 of adapter 200. The attachment/detachment button 272 is biased by a biasing member (not shown) that is disposed within or around stem 273, to an un-actuated condition. Button 272 includes a lip or ledge that is configured to snap behind a corresponding lip or ledge of connecting portion 20 of the surgical instrument 100. As also discussed in WO 2016/057225 A1, the adapter 200 may further include a lock mechanism 280 for fixing the axial position of distal drive member 248. As can be seen in FIG. 21, for example, lock mechanism 280 includes a button 282 that is slidably supported on outer knob housing 202. Lock button 282 is connected to an actuation bar (not shown) that extends longitudinally through outer tube 206. Actuation bar moves upon a movement of lock button 282. In operation, in order to lock the position and/or orientation of distal drive member 248, a user moves lock button 282 from a distal position to a proximal position, thereby causing the lock out (not shown) to move proximally such that a distal face of the lock out moves out of contact with camming member 288, which causes camming member 288 to cam into recess 249 of distal drive member 248. In this manner, distal drive member 248 is prevented from distal and/or proximal movement. When lock button 282 is moved from the proximal position to the distal position, the distal end of actuation bar moves distally into the lock out (not shown), against the bias of a biasing member (not shown), to force camming member 288 out of recess 249, thereby allowing unimpeded axial translation and radial movement of distal drive member 248.

Returning again to FIG. 6, adapter 200 includes an electrical assembly 290 supported on and in outer knob housing 202 and inner housing assembly 204. Electrical assembly 290 includes a plurality of electrical contact blades 292, supported on a circuit board 294, for electrical connection to pass-through connector of plate assembly of shell housing 10 of surgical instrument 100. Electrical assembly 290 serves to allow for calibration and communication information (i.e., life-cycle information, system information, force information) to pass to the circuit board of surgical instrument 100 via an electrical receptacle portion of the power-pack core assembly 106 of surgical instrument 100. Electrical assembly 290 further includes a strain gauge 296 that is electrically connected to circuit board 294. Strain gauge 296 is mounted within the inner housing assembly 204 to restrict rotation of the strain gauge 296 relative thereto. First rotatable proximal drive shaft 212 extends through strain gauge 296 to enable the strain gauge 296 to provide a closed-loop feedback to a firing/clamping load exhibited by first rotatable proximal drive shaft 212. Electrical assembly 290 also includes a slip ring 298 that is non-rotatably and slidably disposed along drive coupling nut 244 of outer tube 206. Slip ring 298 is in electrical connection with circuit board 294 and serves to permit rotation of first rotatable proximal drive shaft 212 and axial translation of drive coupling nut 244 while still maintaining electrical contact of slip ring 298 with at least another electrical component within adapter 200, and while permitting the other electrical components to rotate about first rotatable proximal drive shaft 212 and drive coupling nut 244.

Still referring to FIG. 6, inner housing assembly 204 includes a hub 205 that has a distally oriented annular wall 207 that defines a substantially circular outer profile. Hub 205 includes a substantially tear-drop shaped inner recess or bore that is shaped and dimensioned to slidably receive articulation bearing assembly 252 therewithin. Inner housing assembly 204 further includes a ring plate 254 that is secured to a distal face of distally oriented annular wall 207 of hub 204a. Ring plate 254 defines an aperture 254a therethrough that is sized and formed therein so as to be aligned with second proximal drive shaft 214 and to rotatably receive a distal tip thereof. In this manner, the distal tip of the second proximal drive shaft 214 is supported and prevented from moving radially away from a longitudinal rotational axis of second proximal drive shaft 214 as second proximal drive shaft 214 is rotated to axially translate articulation bearing assembly 252.

Turning next to FIG. 10, in one example, the end effector 500 may be configured for a single use ("disposable loading unit - DLU") and be similar to those DLU's disclosed in U.S. Patent Application Publication No. US 2010/0301097, entitled LOADING UNIT HAVING DRIVE ASSEMBLY LOCKING MECHANISM, U.S. Patent Application Publication No. US 2012/0217284, entitled LOCKING MECHANISM FOR USE WITH LOADING UNITS, and U.S. Patent Application Publication No. US 2015/0374371, entitled ADAPTER ASSEMBLIES FOR INTERCONNECTING SURGICAL LOADING UNITS AND HANDLE ASSEMBLIES, the entire disclosures of each such references being hereby incorporated by reference herein. It is also contemplated that the end effector 500 may be configured for multiple uses (MULU) such as those end effectors disclosed in US Patent Application Publication No. US 2017/0095250, entitled MULTI-USE LOADING UNIT, the entire disclosure of which is hereby incorporated by reference herein.

The depicted surgical instrument 100 fires staples, but it may be adapted to fire any other suitable fastener such as clips and two-part fasteners. In the illustrated arrangement, the end effector 500 comprises a loading unit 510. The loading unit 510 comprises a proximal body portion 520 and a tool assembly 600. Tool assembly 600 includes a pair of jaw members including a first jaw member 610 that comprises an anvil assembly 612 and a second jaw member 700 that comprises a cartridge assembly 701. One jaw member is pivotal in relation to the other to enable the clamping of tissue between the jaw members. The cartridge assembly 701 is movable in relation to anvil assembly 612 and is movable between an open or unclamped position and a closed or approximated position. However, the anvil assembly 612, or both the cartridge assembly 701 and the anvil assembly 612, can be movable.

The cartridge assembly 701 has a cartridge body 702 and in some instances a support plate 710 that are attached to a channel 720 by a snap-fit connection, a detent, latch, or by another type of connection. The cartridge assembly 701 includes fasteners or staples 704 that are movably supported in a plurality of laterally spaced staple retention slots 706, which are configured as openings in a tissue contacting surface 708. Each slot 706 is configured to receive a fastener or staple therein. Cartridge body 702 also defines a plurality of cam wedge slots which accommodate staple pushers 709 and which are open on the bottom (i.e., away from tissue-contacting surface) to allow an actuation sled 712 to pass longitudinally therethrough. The cartridge assembly 701 is removable from channel 720 after the staples have been fired from cartridge body 702. Another removable cartridge assembly is capable of being loaded onto channel 720, such that surgical instrument 100 can be actuated again to fire additional fasteners or staples. Further details concerning the cartridge assembly may be found, for example, in US Patent Application Publication No. US2017/0095250 as well as various other references that have been incorporated by reference herein.

Cartridge assembly 701 is pivotal in relation to anvil assembly 612 and is movable between an open or unclamped position and a closed or clamped position for insertion through a cannula of a trocar. Proximal body portion 520 includes at least a drive assembly 540 and an articulation link 560. In one arrangement, drive assembly 540 includes a flexible drive beam 542 that has a distal end 544 and a proximal engagement section 546. A proximal end of the engagement section 546 includes diametrically opposed inwardly extending fingers 547 that engage a hollow drive member 548 to fixedly secure drive member 548 to the proximal end of beam 542. Drive member 548 defines a proximal porthole which receives connection member 247 of drive tube 246 of first drive converting assembly 240 of adapter 200 when the end effector 500 is attached to the distal end of the adapter 200.

End effector 500 further includes a housing assembly 530 that comprises an outer housing 532 and an inner housing 534 that is disposed within outer housing 532. First and second lugs 536 are each disposed on an outer surface of a proximal end 533 of outer housing 532 and are configured to operably engage the distal end of the adapter 200 as discussed in further detail in WO 2016/057225 A1.

With reference to FIG. 10, for example, anvil assembly 612 includes an anvil cover 630 and an anvil plate 620, which includes a plurality of staple forming depressions. Anvil plate 620 is secured to an underside of anvil cover 630. When tool assembly 600 is in the approximated position, staple forming depressions are positioned in juxtaposed alignment with staple receiving slots of the cartridge assembly 701.

The tool assembly 600 includes a mounting assembly 800 that comprises an upper mounting portion 810 and a lower mounting portion 812. A mounting tail 632 protrudes proximally from a proximal end 631 of the anvil cover 630. A centrally-located pivot member 814 extends from each upper and lower mounting portions 810 and 812 through openings 822 that are formed in coupling members 820. In at least one arrangement, the pivot member 814 of the upper mounting portion 810 also extends through an opening 634 in the mounting tail 632 as well. Coupling members 820 each include an interlocking proximal portion 824 that is configured to be received in corresponding grooves formed in distal ends of the outer housing 532 and inner housing 534. Proximal body portion 520 of end effector 500 includes articulation link 560 that has a hooked proximal end 562. The articulation link 560 is dimensioned to be slidably positioned within a slot in the inner housing. A pair of H-block assemblies 830 are positioned adjacent the distal end of the outer housing 532 and adjacent the distal end 544 of axial drive assembly 540 to prevent outward buckling and bulging of the flexible drive beam 542 during articulation and firing of surgical stapling apparatus 10. Each H-block assembly 830 includes a flexible body 832 which includes a proximal end fixedly secured to the distal end of the outer housing 532 and a distal end that is fixedly secured to mounting assembly 800. In one arrangement, a distal end 564 of the articulation link is pivotally pinned to the right H block assembly 830. Axial movement of the articulation link 560 will cause the tool assembly to articulate relative to the body portion 520.

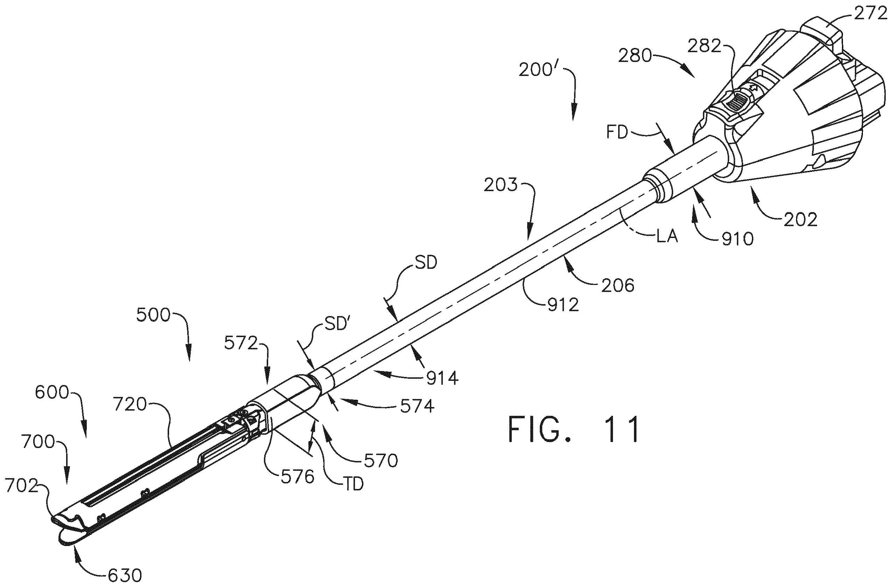

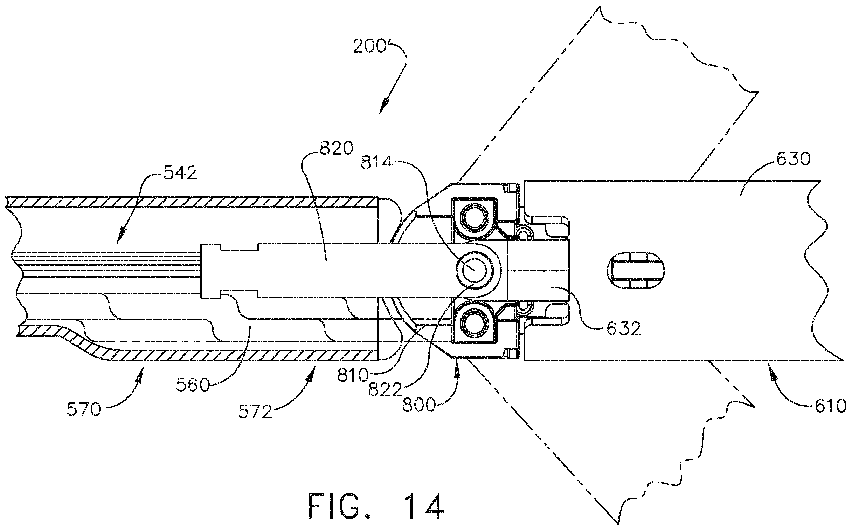

FIGS. 11-15 illustrate an adapter 200' that is substantially identical to adapter 200 described above, except for the differences noted below. As can be seen in FIG. 11, the adapter 200' includes an outer tube 206 that has a proximal end portion 910 that has a first diameter "FD" and is mounted within the outer knob housing 202. The proximal end portion 910 may be coupled to the inner housing assembly 204 or otherwise supported therein in the manners discussed in further detail in WO 2016/057225 A1 for example. The proximal end portion 910 extends proximally from a central tube portion 912 that has a second diameter "SD". In the illustrated embodiment, an end effector 500 is coupled to a distal end 914 of a shaft assembly 203 or outer tube 206. The outer tube 206 defines a longitudinal axis LA that extends between the proximal end portion 910 and the distal end 914 as can be seen in Fig. 11. As can be seen in FIGS. 10 and 11, an outer sleeve 570 of the proximal body portion 520 of the end effector 500 has a distal end portion 572 and a proximal end portion 574. The proximal end portion 574 has a diameter SD' that is approximately equal to the second diameter SD of the central tube portion 912. The distal end portion 572 has a third diameter "TD". In one arrangement, FD and TD are approximately equal and greater than SD. Other arrangements are contemplated wherein FD and TD are not equal, but each are greater than SD. However, it is preferable that for most cases FD and TD are dimensioned for endoscopic insertion through a typical trocar port, cannula or the like. In at least one arrangement (FIG. 11), the outer sleeve 570 is formed with a flat or scalloped side 576 to facilitate improved access within the patient while effectively accommodating the various drive and articulation components of the adapter 200'. In addition, by providing the central tube portion 912 with a reduced diameter may afford the adapter 200' with improved thoracic in-between rib access.

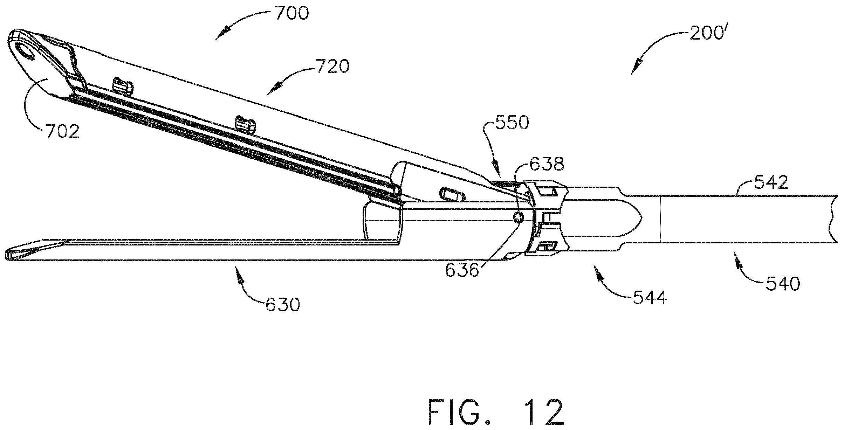

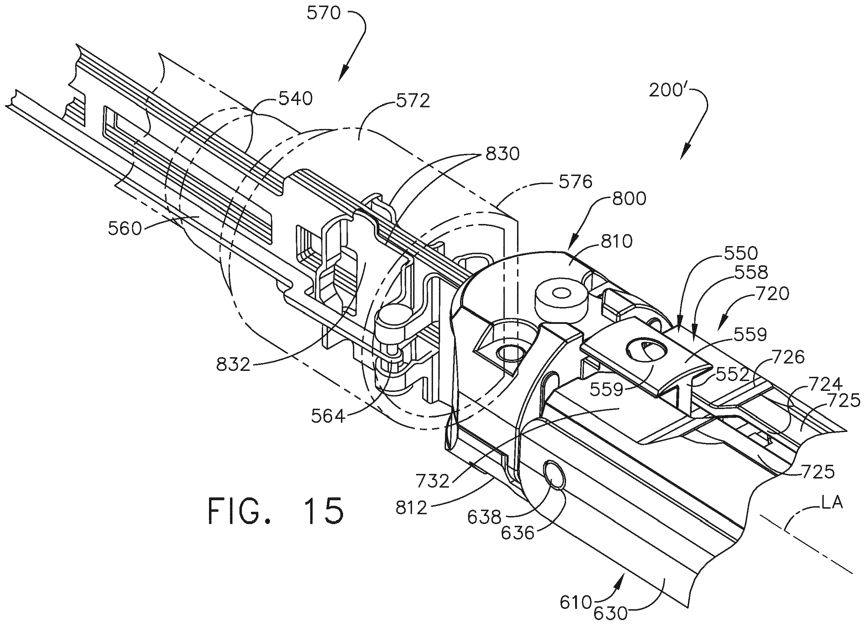

In at least one arrangement, channel 720, which may be machined or made of sheet metal, includes a pair of proximal holes 722 (FIG. 10) that are configured to align with a pair of corresponding holes 636 in the anvil cover 630 to receive corresponding pins or bosses 638 (FIG. 12) to facilitate a pivotal relationship between anvil assembly 612 and cartridge assembly 701. In the illustrated example, a dynamic clamping assembly 550 is attached to or formed at the distal end 544 of the flexible drive beam 542. The dynamic clamping assembly 550 includes a vertical body portion 552 that has a tissue cutting surface 554 formed thereon or attached thereto. See Fig. 10, for example. An anvil engagement feature 556 is formed on one end of the body portion 552 and comprises an anvil engagement tab 557 that protrudes from each lateral side of the body portion 552. Similarly, a channel engagement feature 558 is formed on the other end of the of the body portion 552 and comprises a channel engagement tab 559 that protrudes from each lateral side of the body portion 552. See FIG. 15.

As indicated above, the anvil assembly 612 includes an anvil plate 620. The anvil plate 620 includes an elongate slot 622 that is configured to accommodate the body portion 552 of the dynamic clamping assembly 550 as the dynamic clamping assembly 550 is axially advanced during the firing process. The elongate slot 622 is defined between two anvil plate ledges 624 that extend along each lateral side of the elongate slot 622. See FIG. 10. As the dynamic clamping assembly 550 is distally advanced, the anvil engagement tabs 557 slidably engage the anvil plate ledges 624 to retain the anvil assembly 612 clamped onto the target tissue. Similarly, during the firing operation, the body portion 552 of the dynamic clamping assembly 550 extends through a central slot in the channel 720 and the channel engagement tabs 559 slidably engage channel ledges 725 extending along each side of the central channel slot to retain the cartridge assembly 701 clamped onto the target tissue.

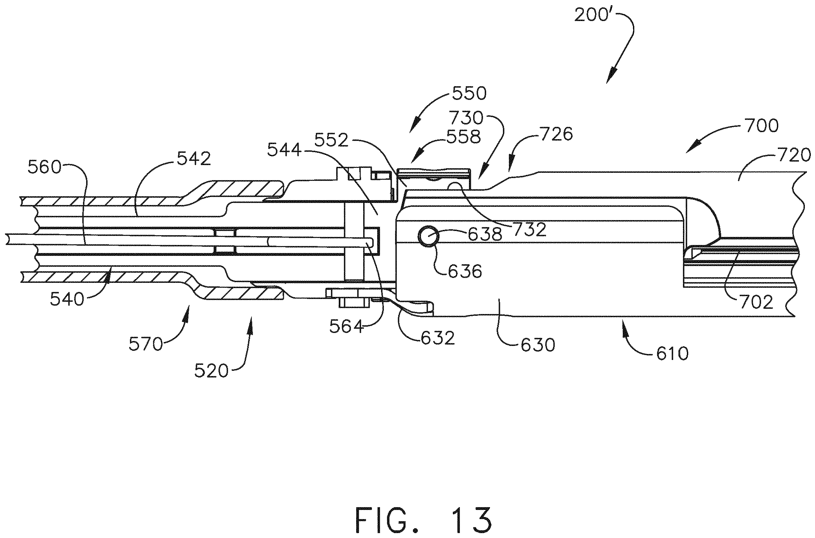

Turning to FIGS. 13 and 15, the channel 720 defines a docking area generally designated as 730 that is configured to accommodate the dynamic clamping assembly 550 when it is in its proximal most position referred to herein as an unfired or starting position. In particular, the docking area 730 is partially defined by planar docking surfaces 732 that provides clearance between the channel engagement tabs 559 on the dynamic clamping assembly 550 to enable the cartridge assembly 701 to pivot to a fully opened position. A ramped or camming surface 726 extends from a distal end of each of the docking surfaces 732. Ramped surface 726 is engaged by the dynamic clamping assembly 550 in order to move the anvil assembly 612 and the cartridge assembly 701 with respect to one another. Similar camming surface could be provided on the anvil assembly 612 in other embodiments. It is envisioned that ramped surfaces 726 may also facilitate the alignment and/or engagement between channel 720 and support plate 620 and/or cartridge body 702. As the drive assembly 540 is distally advanced (fired), the channel engagement tabs 559 on the dynamic clamping assembly 550 engage the corresponding ramped surfaces 726 to apply a closing motion to the cartridge assembly 701 thus closing the cartridge assembly 701 and the anvil assembly 612. Further distal translation of the dynamic clamping assembly 550 causes the actuation sled 712 to move distally through cartridge body 702, which causes cam wedges 713 of actuation sled 712 to sequentially engage staple pushers 709 to move staple pushers 709 vertically within staple retention slots 706 and eject staples 704 into staple forming depressions of anvil plate 620. Subsequent to the ejection of staples 704 from retention slots 706 (and into tissue), the cutting edge 554 of the dynamic clamping assembly 550 severs the stapled tissue as the tissue cutting edge 554 on the vertical body portion 552 of the dynamic clamping assembly 550 travels distally through a central slot 703 of cartridge body 702. After staples 704 have been ejected from cartridge body 702 and a user wishes to use the same instrument 10 to fire additional staples 704 (or another type of fastener or knife), the user can remove the loading unit 510 from the adapter 200' and replace it with another fresh or unspent loading unit. In an alternative arrangement, the user may simply remove the spent cartridge body 702 and replace it with a fresh unspent or unfired cartridge body 702.

The surgical instrument 100 can include sensor assemblies for detecting various states and/or parameters associated with the operation of the surgical instrument 100. A control circuit or processor can monitor these sensed states and/or parameters and then control the operation of the surgical instrument 100 accordingly. For example, the surgical instrument 100 can monitor the current drawn by the motor driving the first force/rotation transmitting/converting assembly 240 (FIG. 6) in order to control the speed at which the clamping member 550 (FIG. 10) is translated. As another example, the surgical instrument 100 can monitor the gap or distance between the jaw members or the anvil plate 620 (FIG. 10) and the cartridge body 702 (FIG. 10) when the end effector 500 is clamped in order to control the speed at which the clamping member 550 is driven thereafter. These and other sensor assemblies with corresponding logic executed by a control circuit or processor in conjunction with the sensor assemblies are described herebelow.

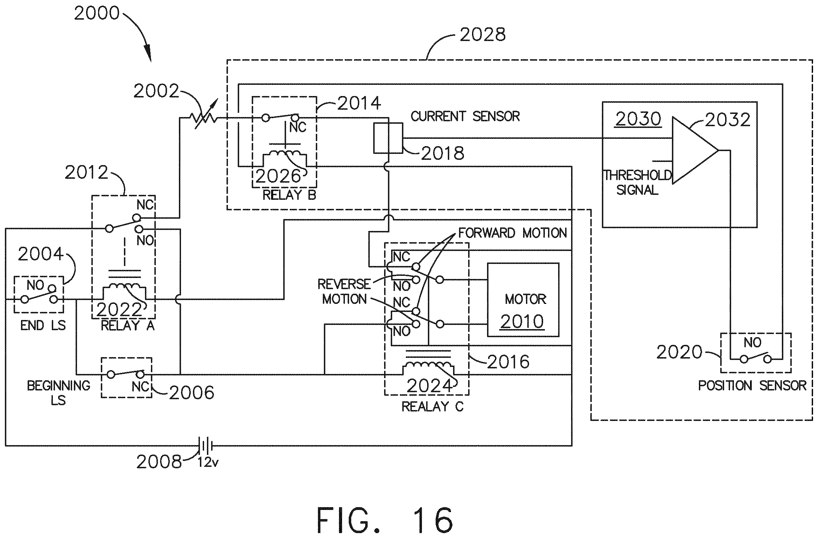

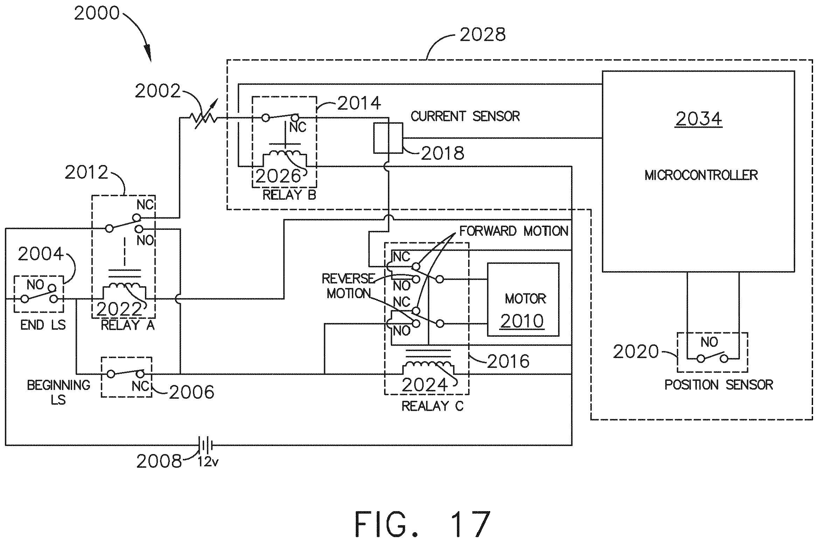

FIGS. 16 and 17 illustrate schematic diagrams a circuit 2000 for controlling a motor 2010 of a surgical instrument, according to various aspects of the present disclosure. In the depicted aspects, the circuit 2000 includes a switch 2002, a first limit switch 2004 (e.g., a normally open switch), a second limit switch 2006 (e.g., a normally closed switch), a power source 2008, and a motor 2010 (e.g., a motor that is configured to drive the first force/rotation transmitting/converting assembly 240). The circuit 2002 can further include a first relay 2012 (e.g., a single-pole double-throw relay), a second relay 2014 (e.g., a single-pole single-throw relay), a third relay 2016 (e.g., a double-pole double-throw relay), a current sensor 2018, and a current detection module 2030. In one aspect, the circuit 2000 can include a motor control circuit 2028 that is configured to sense the electrical current through the motor 2010 and then control the current accordingly. In the aspect depicted in FIG. 16, the second relay 2014, the current sensor 2018, the position sensor 2020, and the current detection module 2030 collectively form the motor control circuit 2028. In the aspect depicted in FIG. 17, the second relay 2014, the current sensor 2018, the position sensor 2020, and the controller 2034 collectively form the motor control circuit 2028. As described below, the motor control circuit 2028 controls the current to the motor 2010 by interrupting the current based upon the sensed current, thus deactivating the motor 2010 when certain conditions occur.

The switch 2002 is activated when an operator of the surgical instrument 100 initiates the firing of the clamping member 550 to clamp the end effector 500 and cut and/or staple tissue. The first limit switch 2004 is configured to remain open when the cutting/stapling operation of the end effector 500 is not yet complete. When the first limit switch 2004 is open, the coil 2022 of first relay 2012 is de-energized, thus forming a conductive path between the power source 2008 and second relay 2014 via a normally-closed contact of relay 2012. The coil 2026 of the second relay 2014 is controlled by the current detection module 2030 and the position sensor 2020 as described below. When the coil 2026 of the second relay 2014 and the coil 2022 of the first relay 2022 are de-energized, a conductive path between the power source 2008 and a normally-closed contact of the third relay 2016 is formed. The third relay 2016 controls the rotational direction of the motor 2010 based on the states of switches 2004, 2006. When first limit switch 2004 is open and the second limit switch 2006 is closed (indicating that the clamping member 550 has not yet fully deployed distally), the coil 2024 of the third relay 2016 is de-energized. Accordingly, when coils 2022, 2024, 2026 are collectively de-energized, current from the power source 2008 flows through the motor 2010 via the normally-closed contacts of the third relay 2016 and causes the forward rotation of the motor 2010, which in turn causes the clamping member 550 to be driven distally by the motor 2010 to clamp the end effector 500 and cut and/or staple tissue.

When the clamping member 550 has been fully advanced distally, the first limit switch 2004 is configured to close. When the first limit switch 2004 is closed, the coil 2022 of the first relay 2012 is energized and the coil 2024 of third relay 2016 is energized via a normally open contact of relay 2012. Accordingly, current now flows to the motor 2010 via normally-open contacts of relays 2012, 2016, thus causing reverse rotation of the motor 2010 which in turn causes the clamping member 550 to retract from its distal position and the first limit switch 2004 to open. The second limit switch 2004 is configured to open when the clamping member 550 is fully retracted. Coil 2022 of relay 2012 remains energized until the second limit switch 2006 is opened, indicating the complete retraction of the clamping member 550.

The magnitude of current through the motor 2010 during its forward rotation is indicative of forces exerted upon the clamping member 550 as it is driven distally by the motor 2010. If a staple cartridge 702 is not loaded into the end effector 500, an incorrect staple cartridge 702 is loaded into the end effector 500, or if the clamping member 550 experiences unexpectedly high resistance from the tissue as it cuts and/or staples the tissue, the resistive force exerted against the clamping member 550 causes an increase in motor torque, which thereby causes the motor current to increase. If the motor current exceeds a threshold, the motor control circuit 2028 can cut off the electrical current to the motor 2010, deactivating the motor and thus pausing the advancement of the clamping member 550. Accordingly, by sensing the current through the motor 2010, the motor control circuit 2028 can differentiate between normal operational thresholds of the deployment of the clamping member 550 and potential error conditions.

The current sensor 2018 may be coupled to a path of the circuit 2000 that conducts current to the motor 2010 during its forward rotation. The current sensor 2018 may be any current sensing device (e.g., a shunt resistor, a Hall effect current transducer, etc.) suitable for generating a signal (e.g., a voltage signal) representative of sensed motor current. The generated signal may be input to the current detection module 2030 for processing therein. According to the aspect depicted in FIG. 16, the current detection module 2030 may be configured for comparing the signal generated by the current sensor 2018 to a threshold signal (e.g., a threshold voltage signal) via a comparator circuit 2032 for receiving the threshold and current sensor 2018 signals and generating a discrete output based on a comparison of the received signals. In some aspects, a value of the threshold signal may be empirically determined *a priori* by measuring the peak signal generated by the current sensor 2018 when the clamping member 550 is initially deployed (e.g., over an initial period or length of its distal movement) during a cutting and stapling operation. In other aspects, the value of the threshold signal can be a pre-determined value that can, in one example, be retrieved from a memory.

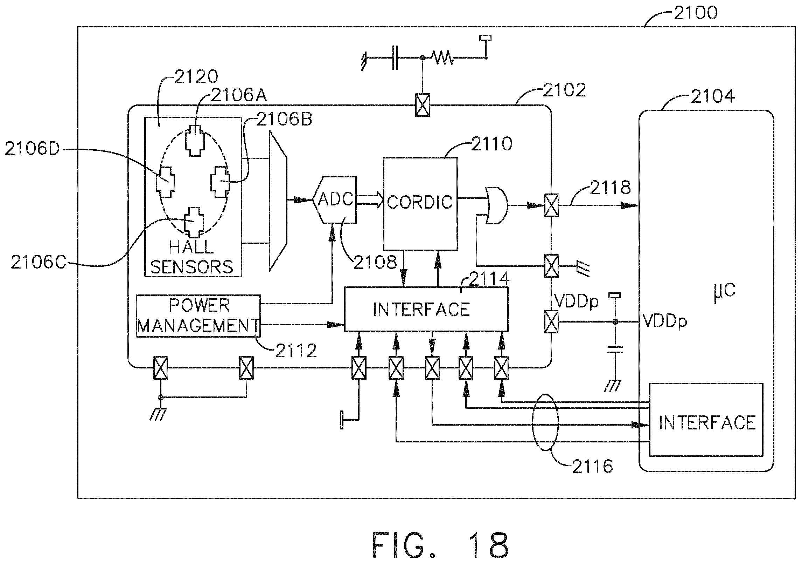

In some aspects, it may be desirable to limit the comparison of the sensed motor current to the threshold value to a particular position or range(s) of positions along the firing stroke of the clamping member 550. In these aspects, the motor control circuit 2028 further includes a position sensor 2020 that is configured to generate a signal indicative of the position of the clamping member 550 (or alternatively, a component of the second or third force/rotation transmitting/converting assemblies 250, 260 for aspects wherein the motor 2010 represented in FIGS. 16 and 17 drives the second or third force/rotation transmitting/converting assemblies
250, 260). The position sensor 2020 can include, for example, the position sensing assembly depicted in FIG. 18 and described in fuller detail below. The position sensor 2020 is connected in series with the comparator circuit 2032 (or the microcontroller 2034 of the aspect depicted in FIG. 17) to limit the comparison based on the position of the clamping member 550. Accordingly, if the signal generated by the current sensor 2018 exceeds the threshold signal (indicating that unexpectedly high resistance is being encountered by the clamping member 550) and the clamping member 550 is within a particular zone as determined by the position sensor 2020, the coil 2026 of the second relay 2014 will be energized. This causes normally-closed switch of the second relay 2014 to open, thereby interrupting current flow to the motor 2010 and pausing the advancement of the clamping member 550. In this way, if the threshold signal is exceeded when the position of the clamping member 550 is not at a position that activates the position sensor 2020, then the motor control circuit 2038 will not deactivate the motor 2010, regardless of the result of the comparison. In other aspects, the motor control circuit 2038 is configured to monitor the motor current along the entirety of the firing stroke of the clamping member 550. In these aspects, the motor control circuit 2038 lacks the position sensor 2020 (or the position sensor 2020 is deactivated) and the output of the comparator circuit 2032 (or the microcontroller 2034) is fed directly to the second relay 2014. Accordingly, if the signal generated by the current sensor 2018 exceeds the threshold signal at any point along the firing stroke of the clamping member 550, then current flow to the motor 2010 is interrupted, in the manner described above.

According to the aspect depicted in FIG. 17, the motor control circuit 2028 can include a processor-based microcontroller 2034 in lieu of the current detection module 2030 described above. Although not shown for purposes of clarity, the microcontroller 2034 may include components well known in the microcontroller art such as, for example, a processor, a random access memory (RAM) unit, an erasable programmable read-only memory (EPROM) unit, an interrupt controller unit, timer units, analog-to-digital conversion (ADC) and digital-to-analog conversion (DAC) units, and a number of general input/output (I/O) ports for receiving and transmitting digital and analog signals. In on example, the microcontroller 2034 includes motor controllers comprising A3930/31 K motor drivers from Allegro Microsystems, Inc. The A3930/31K motor drivers are designed to control a 3-phase brushless DC (BLDC) motor with N-channel external power MOSFETs, such as the motors 152, 154, 156 (FIG. 4). Each of the motor controllers is coupled to a main controller disposed on the main controller circuit board 142b (FIG. 4). The main controller is also coupled to memory, which is also disposed on the main controller circuit board 142b (FIG. 4). In one example, the main controller comprises an ARM Cortex M4 processor from Freescale Semiconductor, Inc. which includes 1024 kilobytes of internal flash memory. The main controller communicates with the motor controllers through an FPGA, which provides control logic signals. The control logic of the motor controllers then outputs corresponding energization signals to their respective motors 152, 154, 156 using fixed frequency pulse width modulation (PWM).

The current sensor 2018 and the position sensor 2020 may be connected to analog and digital inputs, respectively, of the microcontroller 2034, and the coil 2026 of the second relay 2014 may be connected to a digital output of the microcontroller 2034. It will be appreciated that in aspects in which the output of the position sensor 2020 is an analog signal, the position sensor 2020 may be connected to an analog input instead. Additionally, although the circuit 2000 includes relays 2012, 2014, 2016, it will be appreciated that in other aspects the relay switching functionality may be replicated using solid state switching devices, software, and combinations thereof. In certain aspects, for example, instructions stored and executed in the microcontroller 2034 may be used to control solid state switched outputs of the microcontroller 2034. In such aspects, switches 2004, 2006 may be connected to digital inputs of the microcontroller 2034.

FIG. 18 illustrates a schematic diagram of a position sensor 2102 of a surgical instrument 100, according to one aspect of the present disclosure. The position sensor 2102 may be implemented as an AS5055EQFT single-chip magnetic rotary position sensor available from Austria Microsystems, AG. The position sensor 2102 is interfaced with the controller 2104 to provide an absolute positioning system 2100. The position sensor 2102 is a low-voltage and low-power component and includes four Hall effect elements 2106A, 2106B, 2106C, 2106D in an area 2120 of the position sensor 2102 that is located above a magnet that is coupled to a component of the surgical instrument 100. The magnet can be coupled to, for example, a drive shaft of the motor driving the first force/rotation transmitting/converting assembly 240, the proximal drive shaft 212 of the first force/rotation transmitting/converting assembly 240, or a gear assembly that is rotatably driven by the clamping member 550 as the clamping member 550 is translated. In other words, the magnet can be coupled to a component of the surgical instrument 100 such that the angular position of the magnet with respect to the Hall effect elements 2106A, 2106B, 2106C, 2106D corresponds to a longitudinal position of, for example, the clamping member 550. A high-resolution ADC 2108 and a smart power management controller 2112 are also provided on the chip. A CORDIC processor 2110 (for Coordinate Rotation Digital Computer), also known as the digit-by-digit method and Volder's algorithm, is provided to implement a simple and efficient algorithm to calculate hyperbolic and trigonometric functions that require only addition, subtraction, bitshift, and table lookup operations. The angle position, alarm bits, and magnetic field information are transmitted over a standard serial communication interface such as an SPI interface 2114 to the controller 2104. The position sensor 2102 provides 12 or 14 bits of resolution. The position sensor 2102 may be an AS5055 chip provided in a small QFN 16-pin 4x4x0.85mm package.

The Hall effect elements 2106A, 2106B, 2106C, 2106D are located directly above the rotating magnet (not shown). The Hall effect is a well-known effect and for expediency will not be described in detail herein; however, generally, the Hall effect produces a voltage difference (the Hall voltage) across an electrical conductor transverse to an electric current in the conductor and a magnetic field perpendicular to the current. A Hall coefficient is defined as the ratio of the induced electric field to the product of the current density and the applied magnetic field. It is a characteristic of the material from which the conductor is made, since its value depends on the type, number, and properties of the charge carriers that constitute the current. In the AS5055 position sensor 2102, the Hall effect elements 2106A, 2106B, 2106C, 2106D are
capable producing a voltage signal that is indicative of the absolute position of the magnet 1202 in terms of the angle over a single revolution of the magnet 1202. This value of the angle, which is unique position signal, is calculated by the CORDIC processor 2110 is stored onboard the AS5055 position sensor 2102 in a register or memory. The value of the angle that is indicative of the position of the magnet 1202 over one revolution is provided to the controller 2104 in a variety of techniques, for example, upon power up or upon request by the controller 2104.

The AS5055 position sensor 2102 requires only a few external components to operate when connected to the controller 2104. Six wires are needed for a simple application using a single power supply: two wires for power and four wires 2116 for the SPI interface 2114 with the controller 2104. A seventh connection can be added in order to send an interrupt to the controller 2104 to inform that a new valid angle can be read. Upon power-up, the AS5055 position sensor 2102 performs a full power-up sequence including one angle measurement. The completion of this cycle is indicated as an INT output 2118, and the angle value is stored in an internal register. Once this output is set, the AS5055 position sensor 2102 suspends to sleep mode. The controller 2104 can respond to the INT request at the INT output 2118 by reading the angle value from the AS5055 position sensor 2102 over the SPI interface 2114. Once the angle value is read by the controller 2104, the INT output 2118 is cleared again. Sending a "read angle" command by the SPI interface 2114 by the controller 2104 to the position sensor 2102 also automatically powers up the chip and starts another angle measurement. As soon as the controller 2104 has completed reading of the angle value, the INT output 2118 is cleared and a new result is stored in the angle register. The completion of the angle measurement is again indicated by setting the INT output 2118 and a corresponding flag in the status register.

Due to the measurement principle of the AS5055 position sensor 2102, only a single angle measurement is performed in very short time (∼600µs) after each power-up sequence. As soon as the measurement of one angle is completed, the AS5055 position sensor 2102 suspends to power-down state. An on-chip filtering of the angle value by digital averaging is not implemented, as this would require more than one angle measurement and, consequently, a longer power-up time that is not desired in low-power applications. The angle jitter can be reduced by averaging of several angle samples in the controller 2104. For example, an averaging of four samples reduces the jitter by 6dB (50%).

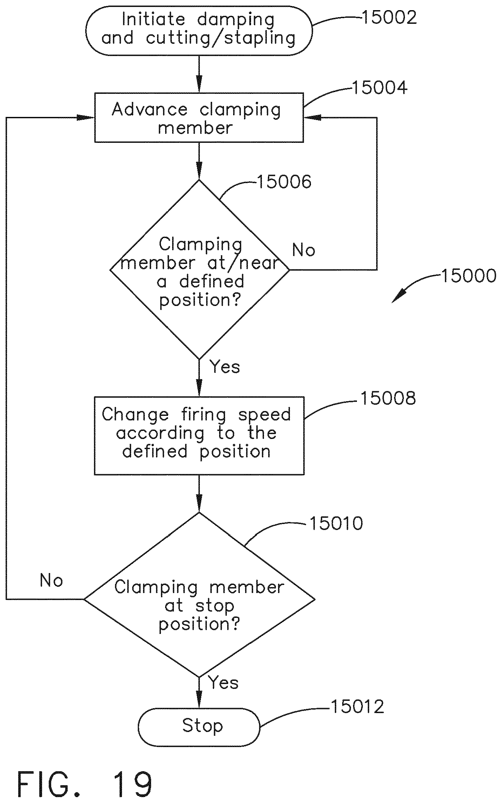

FIG. 19 illustrates a logic flow diagram of a process 15000 for controlling a speed of a clamping member 550 during a firing stroke, according to one aspect of the present disclosure. In the following description of the process 15000, reference should also be made to FIGS. 102-104, which depict various sensor assemblies utilized by the process 15000, and FIG. 21, which depicts various firing strokes of the clamping member 550 executed according to the process 15000. The presently described process 15000 can be executed by a controller, which includes the control circuit depicted in FIGS. 102-103, the microcontroller 2104 of FIG. 104, or another control circuit and/or processor that is executing logic and/or instructions stored in a memory of the surgical instrument 100. The process 15000 begins to be executed when the clamping and cutting/stapling operations of the end effector 500 are initiated 15002.

Accordingly, the process 15000 executed by the controller advances 15004 the clamping member 550 from a first or proximal position by energizing the motor 2010 to which the clamping member 550 is operably connected. The advancement of the clamping member 550 between a first or proximal position and a second or distal position can be referred to as a stroke or a firing stroke. During the course of a full stroke of the clamping member 550, the clamping member 550 will clamp the end effector 500 and then cut and/or staple tissue held thereby. The stroke of the clamping member 550 can be represented, for example, as a graph where the x-axis corresponds to the distance or time over which the clamping member 550 has advanced, as depicted in FIG. 21. The actions effectuated by the clamping member 550 can correspond to positions or zones defined within the stroke of the clamping member 550. For example, there can be a position in the stroke where the clamping member 550 has closed the end effector 500 and is thereafter cutting and/or stapling tissue. As another example, there can be a position in the stroke of the clamping member 550 where the clamping member 550 is no longer ejecting staples or cutting tissue. The controller can also take various actions according to the position of the clamping member 550. For example, there can be a position where the speed at which the clamping member 550 is driven is controlled or changed by a controller. These positions or zones can refer to actual physical positions at which the clamping member is located or relative positions within the stroke of the clamping member. The positions or zones can alternatively be represented as times in the stroke of the clamping member 550.

As the clamping member 550 is advanced 15004, the controller determines 15006 whether the clamping member 550 is at or near (i.e., within a tolerance of) a defined position in the firing stroke of the clamping member 550. A defined position is a pre-defined location in the firing stroke of the clamping member 550 where the controller is configured to increase the clamping member speed (i.e., step up the motor 2010) or decrease the clamping member speed (i.e., step down the motor 2010). There can be zero, one, or multiple defined positions in the process 15000 executed by the controller. The defined positions can be located at or near the proximal or distal ends of the firing stroke or can be located at any intermediate position therebetween. In some aspects, the closure end position and the firing end position are defined positions wherein the controller can be configured, for example, to slow the speed at which the clamping member 550 is being driven by the motor 2010. The closure end position corresponds to the location in the firing stroke of the clamping member 550 after the clamping member 550 has closed the end effector 500 and is thereafter cutting tissue and/or firing staples. In one example, slowing the clamping member 550 as it approaches the closure end position can be useful in order to prevent the clamping member 550 from inadvertently colliding with a lockout stop when there is no staple cartridge present in the end effector 500. The firing end position corresponds to the distal point reached by the clamping member 550 in its firing stroke to cut tissue and/or fire staples from the end effector 500. In one example, slowing the clamping member 550 as it approaches the firing end position can be useful in order to prevent the clamping member 550 from inadvertently colliding with the distal end of the anvil elongated slot 622. In yet another aspect, the firing stroke of the clamping member 550 includes an intermediate defined position positioned between the closure end position and the firing end position where the controller is configured to drive the clamping member 550 at a faster speed.

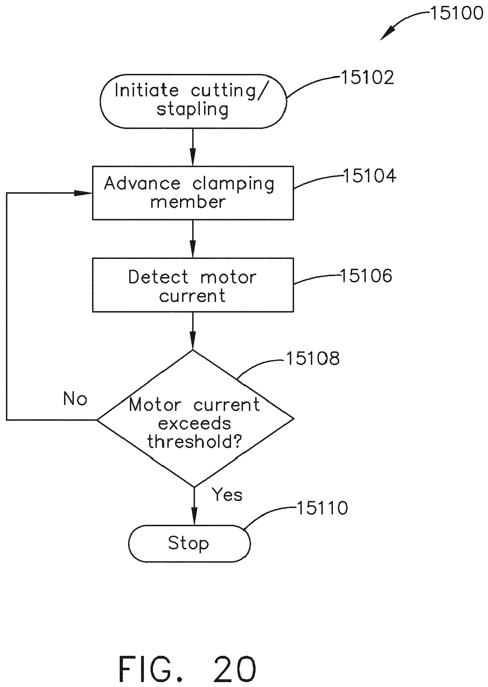

In some aspects, the controller determines 15006 whether the clamping member 550 is approaching or located at a defined position by detecting the present position of the clamping member 550 (e.g., via the position sensor 2102), retrieving one or more stored positions from a memory, and then comparing the detected position to the one or more stored positions to determine if the detected position matches or is within a tolerance distance from at least one of the stored positions. In other aspects, the controller determines 15006 whether the clamping member 550 is approaching or located at a defined position by sensing the motor current and determining whether the sensed motor current or the rate of change of the sensed motor current has exceeded a particular threshold (as described below in FIG. 20). If the controller determines 15006 that the clamping member 550 is not located at a defined position, the process 15000 proceeds along the NO branch and loops back to continue advancing 15004 the clamping member 550.

If the controller determines 15006 that the clamping member 550 is located at (or within a tolerance of) a defined position, the process 15000 proceeds along the YES branch and then changes 15008 the clamping member speed in the manner dictated by the particular defined position. When the controller determines 15006 that the clamping member 550 is located at or near a defined position, the controller can retrieve the manner in which the clamping member speed is to be changed (e.g., whether the speed is to be increased or decreased, a particular value or speed range to which the speed is to be set, or a function for calculating a value or speed range to which the speed is to be set) from a memory that stores each how the clamping member speed is to be changed in association with each of the stored positions. The controller then controls the motor 2010 to increase or decrease the speed at which the clamping member 550 is driven accordingly.

The controller then determines 15010 whether the clamping member 550 is located at a stop position. The controller can determine 15010 the location of the clamping member 550 in the same manner described above, namely via a position sensor 2102 or sensing the motor current relative to one or more thresholds. If the clamping member 550 is located at the stop position, then the process 15000 proceeds along the YES branch and stops 15012. When the process 15000 executed by the controller stops 15012, the controller can take various actions, such as cutting the current to the motor 2010 or displaying a notification to the user that the clamping member 550 has stopped. If the clamping member 550 is not located at the stop position, then the process 15000 proceeds along the NO branch and continues to advance 15004 the clamping member 552. This loop continues until the clamping member 550 reaches the stop position and the process 15000 stops 15012.

FIG. 20 illustrates a logic flow diagram of a process 15100 for detecting a defined position according to motor current, according to one aspect of the present disclosure. In the following description of the process 15100, reference should also be made to FIGS. 102-104, which depict various sensor assemblies utilized by the process 15100, and FIG. 21, which depicts various firing strokes of the clamping member 550 executed according to the process 15100. The presently described process 15100 can be executed by a controller, which includes the control circuit depicted in FIGS. 102-103, the microcontroller 2104 of FIG. 104, or another control circuit and/or processor that is executing logic and/or instructions stored in a memory of the surgical instrument 100. The process 15100 begins to be executed when the cutting/stapling operation of the end effector 500 is initiated 15102.

As the controller controls the motor 2010 to advance 15104 the clamping member 550, the controller monitors or detects 15106 the motor current (e.g., via the current sensor 2018). Further, the controller determines 15108 whether the detected motor current or the rate of change of the detected motor current is greater than or equal to a particular threshold value that is indicative of the firing end position in the stroke of the clamping member 550. In other words, the controller determines 15108 whether the motor current spikes or peaks above a certain level, either by comparing the value of the motor current or the rate of change of the motor current to a particular threshold. Because the motor current tends to sharply increase above a particular threshold as the clamping member 550 approaches certain positions, such as the closure end position and the firing end position, the controller can be configured to monitor the motor current for an indicative increase in the motor current and then take action to slow the clamping member 550 or otherwise prevent the clamping member 550 as it approaches these positions. Slowing the clamping member 550 in this manner can prevent the clamping member 550 from sharply contacting the distal ends of the anvil assembly 610 and/or cartridge assembly 700. In some aspects, the controller can be configured to cross-reference the detected motor current with a position detected from a position sensor 2102 to confirm that the clamping member 550 is located at or near a position where it would be expected for the motor current to increase or decrease in the manner that is being detected. If the controller determines 15108 that the motor current has not exceeded the threshold, the process 15100 proceeds along the NO branch and continues advancing 15104 the clamping member 550. If the controller determines 15108 that the motor current has exceed the end of stroke threshold, the process 15100 proceeds along the YES branch and the process 15100 stops 15110. In some aspects, when the process 15100 stops 15110 the controller de-energizes the motor 2010.

To provide further explanation regarding the function(s) described above that the controller is configured to execute, the processes 15000, 15100 will be discussed in terms of several example firing strokes depicted in FIG. 21. FIG. 21 illustrates a first graph 15200 and a second graph 15202, each of which depict a first firing stroke 15204, a second firing stroke 15206, and a third firing stroke 15208 of the clamping member 550 between an initial or proximal position 15216 and an end or distal position 15218. The first graph 15200 depicts motor current 15203 versus clamping member displacement distance 15201 and the second graph 2302 depicts clamping member speed 15205 versus clamping member displacement distance 15201 for the example firing strokes 15204, 15206, 15206 of the clamping member 550. The displacement distance 15201 axis is delineated into a "CLOSURE" zone, a "CUTTING/STAPLING" zone, and a "STOP" zone, which indicates the action(s) that the clamping member 550 is effectuating in each respective portion of its firing stroke. In combination, the first graph 15200 and the second graph 15202 illustrate the relationship between motor current 15203 and clamping member speed 15205 for different firing strokes 15204, 15206, 15208 and the resulting actions taken by a controller executing the processes 15000, 15100 depicted in FIGS. 19-20.

For each of the firing strokes 15204, 15206, 15208, as the displacement member 550 advances from the initial position 15216 to the closure end position 15210 the motor current sharply increases 15220, 15222, 15224. In one example, the controller executing the process 15000 depicted in FIG. 19 can detect this sharp increase 15220, 15222, 15224 in the motor current by sensing when the rate of change in the motor current for each of the firing strokes 15204, 15206, 15208 exceeds a threshold, as depicted in FIG. 21. The process 15000 then decreases 15226, 15228, 15230 the speed of the clamping member 550 accordingly as it reaches the closure end position 15210. As the clamping member 550 advances past the closure end position 15210, the controller increases 15232, 15234, 15236 the speed at which the clamping member 550 is driven until the clamping member 550 is driven within a particular speed range S₁, S₂, S₃. In one example, the controller increases 15232, 15234, 15236 the speed at which the clamping member 550 is driven until the clamping member 550 is driven within a speed range S₁, S₂, S₃ corresponding to the thickness of the tissue clamped at the end effector 500.

In some aspects, such with the second and third firing strokes 15206, 15208, the clamping member 550 is thereafter driven at a consistent speed or within a consistent speed range. In other aspects, such as with the first firing stroke 15204, the controller is configured to control the motor 2010 to drive the clamping member 550 at varying speeds to account for varying tissue properties. For example, in the first firing stroke 15204 the motor current increases 15238 as the clamping member 550 approaches an intermediate position 15214, potentially due to the cutting surface 554 of the clamping member 550 encountering increasingly thick tissue. In response, the controller can be configured to decrease 15252 the speed at which the clamping member 550 is driven in order to prevent the motor current from continuing to increase. Decreasing 15252 the clamping member speed reduces the current drawn by the motor 2010 because it lowers the torque on the motor 2010. In some aspects, the controller can be configured to change the maximum acceptable current or torque limits on the motor 2010 in response to detected conditions. For example, in the first firing stroke 15204 the first or initial maximum acceptable current limit of the motor 2010 is delineated by the upper bound of the current range i₂ that was selected by the controller in accordance with the clamped tissue thickness. However, when the motor current 15238 increases as the clamping member 550 approaches the intermediate position 15214, the controller can be configured to upwardly adjust the maximum motor current to a second maximum acceptable current limit delineated by the upper bound of the current range i₃.

As the clamping member 550 approaches the firing end position 15212 the motor current sharply increases 15240, 15242, 15244 for each of the firing strokes 15204, 15206, 15208 until it reaches a threshold 15215. The controller executing the process 15000 depicted in FIG. 19 can detect that the motor current has reached or surpassed this threshold 15215, which is indicative of the clamping member 550 approaching the firing end position 15212. The process 15000 then decreases 15246, 15248, 15250 the speed of the clamping member 550 accordingly in each of the firing strokes 15204, 15206, 15208 and the clamping member 550 slows as it reaches the stop position 15218 (i.e., the distal most position of its firing stroke). When the clamping member 550 reaches the stop position 15218, the processes 15000, 15100 can stop and the controller can take various actions, such as displaying an alert to the operator of the surgical instrument 100 indicating that the clamping, cutting, and/or stapling by the surgical instrument 100 has been completed.

In some aspects, the surgical instrument 100 includes stops that are configured to prevent the clamping member 550 (or another component of the firing drive system) from becoming damaged by inadvertently colliding with the anvil assembly 610 and/or the cartridge assembly 700 at the end of its firing stroke. The stops can be constructed from materials that are deformable, bendable, or configured to strain elastically in order to absorb or attenuate the forces from the clamping member 550 as it is advanced to the terminal position of its firing stroke. The stops can be utilized in combination with, or in lieu of, a controller executing a process, such as the process 15100 depicted in FIG. 20, to detect the firing stroke end position and then slow and/or stop the clamping member 550 accordingly.

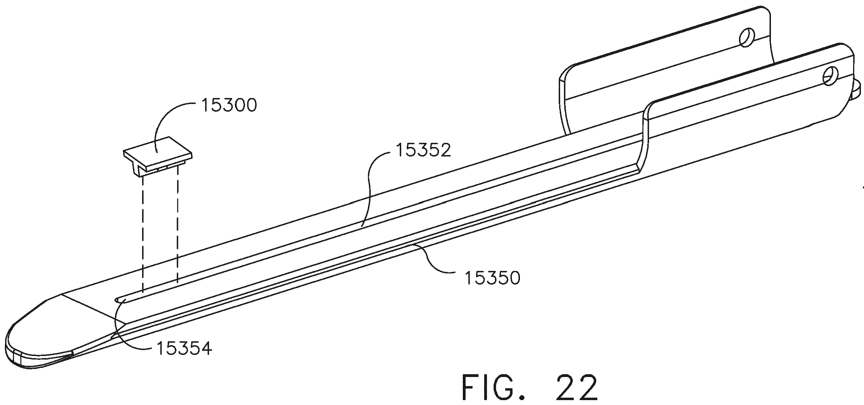

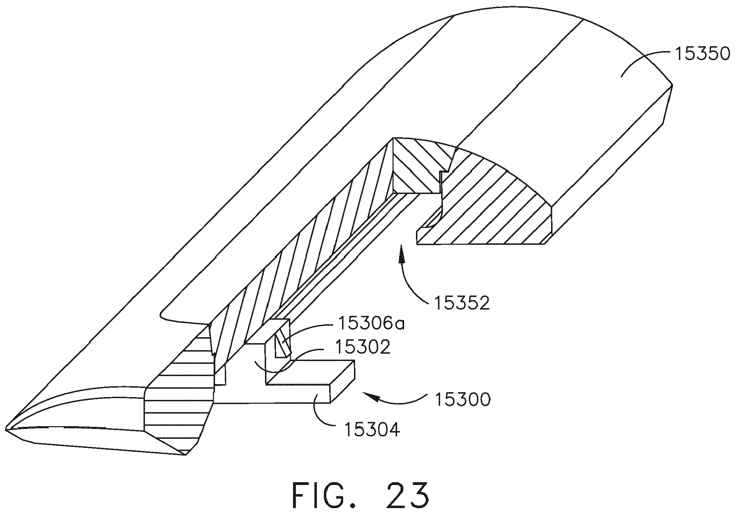

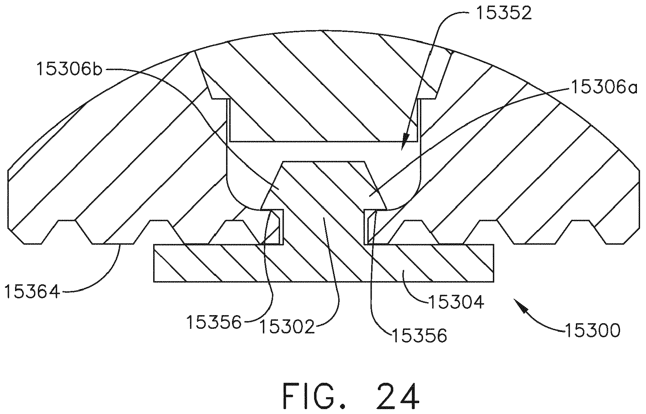

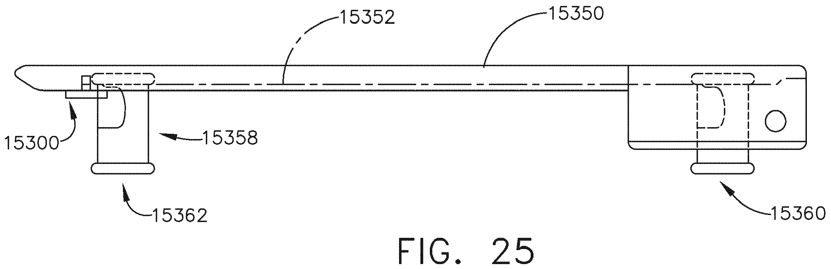

FIGS. 22-25 illustrate various views of a stop member 15300 that is engaged with the elongated slot 15352 of the anvil plate 15350. In the depicted aspect, the stop member 15300 includes a vertical stem or body 15302, a base 15304 extending orthogonally from the body 15302, and one or more flanges 15306a, 15306b. The base 15304 extends across the surface 15364 of the anvil plate 15350. The flanges 15306a, 15306b bear against the interior surface of the shelf 15356 of the elongated slot 15352 and the base 15304 bears against the surface 15364, which secures the stop member 15300 within the elongated slot 15352 and thus prevents the stop member 15300 from being withdrawn therefrom. The stop member 15300 can be positioned at or adjacently to the distal end 15354 of the elongated slot 15352 and serve as a physical obstruction or barrier preventing the clamping member 15358 from colliding with the distal end 15354 during the stroke of the clamping member 15358 as it translates from a first or proximal position 15360 to a second or distal position 15362.

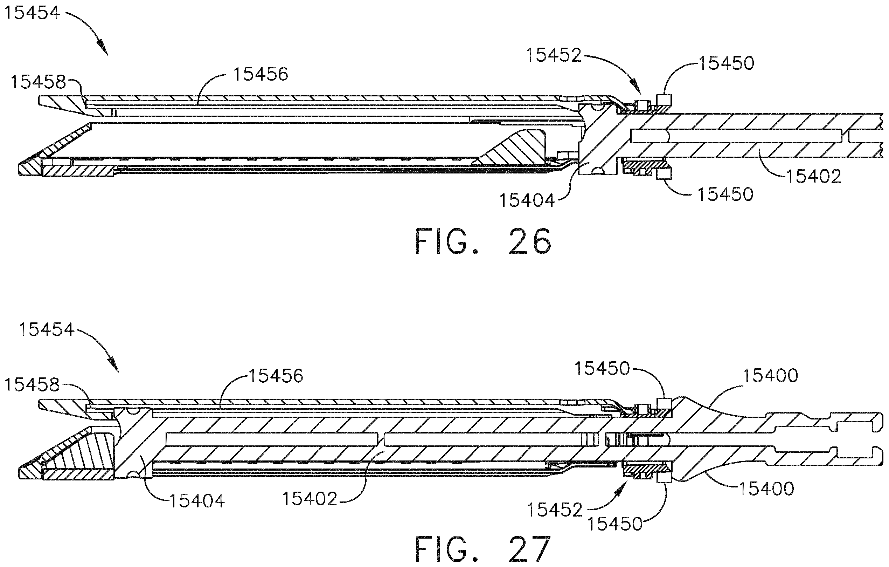

FIGS. 26-27 illustrate longitudinal sectional views of an end effector 15454 and a drive assembly including a stop member 15400. In one aspect, the surgical instrument 100 includes one or more projections 15450 extending from the shaft assembly 203 (FIG. 1) or the end effector 15454. In the depicted aspect, the projections 15450 extend outwardly from the proximal portion of the end effector 15454, adjacent to the pivot joint 15452. The drive beam 15402 includes one or more stop members 15400 extending generally orthogonally therefrom that are configured to contact the projections 15450. The stop members 15400 are rigidly connected to the drive beam 15402 such that the drive beam 15402 is prevented from being advanced further distally when the stop members 15400 contact the projections 15450. The stop members 15400 are positioned on the drive beam 15402 such that the clamping member 15404 does not contact the distal end 15458 of the elongated slot 15456 when the stop members 15400 contact the projections 15450. Stated differently, the distance from the distal end 15458 of the elongated slot 15456 to the projections 15450 is larger than the distance between the distal end of the clamping member 15404 and the stop members 15400. The projections 15450 and/or the stop members 15400 can be constructed from deformable materials or materials that are configured to strain elastically.

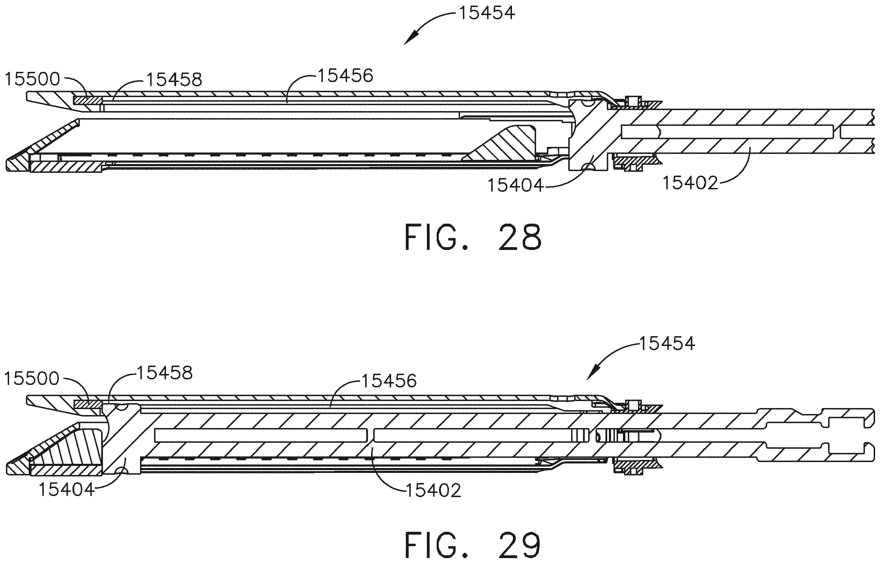

FIGS. 28-29 illustrate longitudinal sectional views of an end effector 15454 including a stop member 15500 located distally in the elongated slot 15456. In the depicted aspect, the end effector 15454 includes a stop member 15500 located at the distal end 15458 of the elongated slot. In this aspect, the stop member 15500 is located within or occupies the distal end 15458 or the distal end 15458 terminates at the stop member 15500. In either case, the stop member 15500 is positioned such that the clamping member 15404 is configured to contact it when the clamping member 15404 has advanced to the most distal position in its firing stroke. The stop member 15500 can be constructed from deformable materials or materials that are configured to strain elastically.

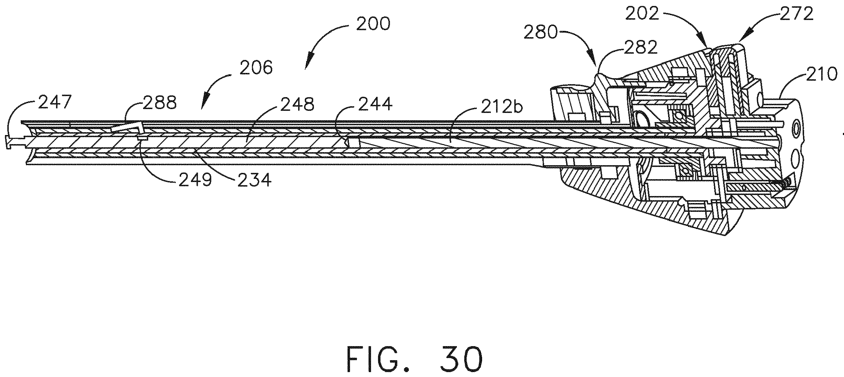

FIG. 30 illustrates a cross-sectional view of the adapter 200. The adapter 200 can include a locking mechanism 280 that is configured to fix the axial or longitudinal position of the distal drive member 248. In some cases, it may be desirable for the lock mechanism 280 to control a switch or transmit a signal to the controller to indicate that the lock mechanism 280 is engaged and thus that the motor 2010 should not be activated to attempt to drive the distal drive member 248. In one aspect, the adapter 200 include a switch (not shown) that is tripped when the camming member 288 of the locking mechanism 280 cams into the recess 249 of the distal drive member 248. The switch is communicably coupled to the controller of the surgical instrument 100. If the controller determines that the locking mechanism switch has indicated that the locking mechanism 280 is engaged, then the controller can limit or cut current to the motor 2010 in order to prevent the motor 2010 from attempting to drive the locked distal drive member 248. Likewise, when the camming member 288 is withdrawn from the recess 249 of the distal drive member 248, then the switch can be un-tripped or re-tripped to indicate to the controller that the locking mechanism 280 has been disengaged and that the motor 2010 can thus be energized to drive the distal drive member 248. Such an arrangement can be useful in order to, for example, prevent damage to the motor 2010 and/or locking mechanism 280.

Although various aspects have been described herein, many modifications and variations to those aspects may be implemented. For example, different types of end effectors may be employed. Also, where materials are disclosed for certain components, other materials may be used. The foregoing description and following claims are intended to cover all such modification and variations.

Any patent, publication, or other disclosure material, in whole or in part, that is said to be incorporated by reference herein is incorporated herein only to the extent that the incorporated materials does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Various aspects of the subject matter described herein are set out in the following numbered examples:
Example 1 - A surgical instrument comprising motor and a current sensor that is configured to sense a current drawn by the motor. The surgical instrument further comprises a control circuit that is coupled to the motor and the current sensor. The control circuit is configured to detect a position of a clamping member that is drivable by the motor between a first position and a second position. In at least one example, the clamping member is configured to transition an end effector to a closed position as the clamping member moves from the first position to the second position and deploy a plurality of staples from a cartridge that is positioned in the end effector after the end effector is in the closed position as the clamping member moves to the second position. The control circuit is further configured to detect whether the current drawn by the motor exceeds a threshold via the current sensor and, upon detecting that the current drawn by the motor exceeds the threshold, control the motor to change a speed at which the clamping member is driven.
Example 2 - The surgical instrument of Example 1, wherein the clamping member is configured to deploy staples from a staple cartridge positioned within the end effector and the current drawn by the motor that exceeds the threshold corresponds to a position of the clamping member wherein the staples have been fully deployed from the staple cartridge.
Example 3 - The surgical instrument of Examples 1 or 2, wherein a knife is coupled to the clamping member and is driven through the end effector as the clamping member moves from the first position to the second position. The current that is drawn by the motor exceeds the threshold that corresponds to a distal position of the knife.
Example 4 - The surgical instrument of Examples 1, 2 or 3, wherein the current drawn by the motor exceeding the threshold corresponds to a position of the clamping member wherein the end effector is in the closed position.
Example 5 - The surgical instrument of Examples 1, 2, 3 or 4, wherein the control circuit is configured to control the motor to decrease the speed at which the clamping member is driven.
Example 6 - The surgical instrument of Examples 1, 2, 3, 4 or 5, wherein the control circuit is configured to detect whether a value of the current drawn by the motor exceeds the threshold.
Example 7 - The surgical instrument of Examples 1, 2, 3, 4, 5 or 6, wherein the control circuit is configured to detect whether a rate of change of the current drawn by the motor exceeds the threshold.
Example 8 - A surgical instrument comprising a motor and a current sensor that is configured to sense a current that is drawn by the motor. A control circuit is coupled to the motor and the current sensor. In at least one example, the control circuit is configured to detect a position of a clamping member that is drivable by the motor between a first position and a second position. In at least one example, the clamping member is configured to transition an end effector to a closed position as the clamping member moves from the first position to the second position and deploy a plurality of staples from a cartridge positioned in the end effector after the end effector is in the closed position as the clamping member moves to the second position. The control circuit is further configured to control the motor to change a speed at which the clamping member is driven at a defined position between the first position and the second position and detect the defined position according to the current drawn by the motor via the current sensor.
Example 9 - The surgical instrument of Example 8, wherein the clamping member is configured to deploy staples from a staple cartridge that is positioned within the end effector and the defined position corresponds to a position of the clamping member wherein the staples have been fully deployed from the staple cartridge.
Example 10 - The surgical instrument of Example 8, wherein the defined position corresponds to a position of the clamping member wherein the end effector is in the closed position.
Example 11 - The surgical instrument of Example 8, wherein the defined position corresponds to an increase in the current drawn by the motor above a threshold.
Example 12 - The surgical instrument of Example 11, wherein the control circuit is configured to detect whether a value of the current drawn by the motor exceeds the threshold.
Example 13 - The surgical instrument of Examples 11 or 12, wherein the control circuit is configured to detect whether a rate of change of the current drawn by the motor exceeds the threshold.
Example 14 - The surgical instrument of Example 8, wherein the control circuit is configured to control the motor to decrease the speed at which the clamping member is driven.

Many of the surgical instrument systems described herein are motivated by an electric motor; however, the surgical instrument systems described herein can be motivated in any suitable manner. In various instances, the surgical instrument systems described herein can be motivated by a manually-operated trigger, for example. In certain instances, the motors disclosed herein may comprise a portion or portions of a robotically controlled system. Moreover, any of the end effectors and/or tool assemblies disclosed herein can be utilized with a robotic surgical instrument system. U.S. Patent Application Serial No. 13/118,241, entitled SURGICAL STAPLING INSTRUMENTS WITH ROTATABLE STAPLE DEPLOYMENT ARRANGEMENTS, now U.S. Patent No. 9,072,535, for example, discloses several examples of a robotic surgical instrument system in greater detail.

The surgical instrument systems described herein have been described in connection with the deployment and deformation of staples; however, the embodiments described herein are not so limited. Various embodiments are envisioned which deploy fasteners other than staples, such as clamps or tacks, for example. Moreover, various embodiments are envisioned which utilize any suitable means for sealing tissue. For instance, an end effector in accordance with various embodiments can comprise electrodes configured to heat and seal the tissue. Also, for instance, an end effector in accordance with certain embodiments can apply vibrational energy to seal the tissue.

Although various devices have been described herein in connection with certain embodiments, modifications and variations to those embodiments may be implemented. Particular features, structures, or characteristics may be combined in any suitable manner in one or more embodiments. Thus, the particular features, structures, or characteristics illustrated or described in connection with one embodiment may be combined in whole or in part, with the features, structures or characteristics of one ore more other embodiments without limitation. Also, where materials are disclosed for certain components, other materials may be used. Furthermore, according to various embodiments, a single component may be replaced by multiple components, and multiple components may be replaced by a single component, to perform a given function or functions. The foregoing description and following claims are intended to cover all such modification and variations.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

While this invention has been described as having exemplary designs, the present invention may be further modified within the spirit and scope of the disclosure. This application is therefore intended to cover any variations, uses, or adaptations of the invention using its general principles.

## Claims

1. A surgical instrument comprising:
a motor;
a current sensor configured to sense a current drawn by the motor; and
a control circuit coupled to the motor and the current sensor, the control circuit configured to:
detect a position of a clamping member drivable by the motor between a first position and a second position, wherein the clamping member is configured to:
transition an end effector to a closed position as the clamping member moves from the first position to the second position; and
deploy a plurality of staples from a cartridge positioned in the end effector after the end effector is in the closed position as the clamping member moves to the second position;
wherein the control circuit is further configured to:
detect whether the current drawn by the motor exceeds a threshold via the current sensor; and
upon detecting that the current drawn by the motor exceeds the threshold, control the motor to change a speed at which the clamping member is driven.

2. The surgical instrument of Claim 1, wherein:
the clamping member is configured to deploy staples from a staple cartridge positioned within the end effector; and
the current drawn by the motor exceeding the threshold corresponds to a position of the clamping member wherein the staples have been fully deployed from the staple cartridge.

3. The surgical instrument of Claim 1, further comprising:
a knife coupled to the clamping member, the knife driven through the end effector as the clamping member moves from the first position to the second position;
wherein the current drawn by the motor exceeding the threshold corresponds to a distal position of the knife.

4. The surgical instrument of Claim 1, wherein the current drawn by the motor exceeding the threshold corresponds to a position of the clamping member wherein the end effector is in the closed position.

5. The surgical instrument of Claim 1, wherein the control circuit is configured to control the motor to decrease the speed at which the clamping member is driven.

6. The surgical instrument of Claim 1, wherein the control circuit is configured to detect whether a value of the current drawn by the motor exceeds the threshold.

7. The surgical instrument of Claim 1, wherein the control circuit is configured to detect whether a rate of change of the current drawn by the motor exceeds the threshold.

8. A surgical instrument comprising:
a motor;
a current sensor configured to sense a current drawn by the motor; and
a control circuit coupled to the motor and the current sensor, the control circuit configured to:
detect a position of a clamping member drivable by the motor between a first position and a second position, wherein the clamping member is configured to:
transition an end effector to a closed position as the clamping member moves from the first position to the second position; and
deploy a plurality of staples from a cartridge positioned in the end effector after the end effector is in the closed position as the clamping member moves to the second position;
wherein the control circuit is further configured to:
control the motor to change a speed at which the clamping member is driven at a defined position between the first position and the second position; and
detect the defined position according to the current drawn by the motor via the current sensor.

9. The surgical instrument of Claim 8, wherein the clamping member is configured to deploy staples from a staple cartridge positioned within the end effector and the defined position corresponds to a position of the clamping member wherein the staples have been fully deployed from the staple cartridge.

10. The surgical instrument of Claim 8, wherein the defined position corresponds to a position of the clamping member wherein the end effector is in the closed position.

11. The surgical instrument of Claim 8, wherein the defined position corresponds to an increase in the current drawn by the motor above a threshold.

12. The surgical instrument of Claim 11, wherein the control circuit is configured to detect whether a value of the current drawn by the motor exceeds the threshold.

13. The surgical instrument of Claim 11, wherein the control circuit is configured to detect whether a rate of change of the current drawn by the motor exceeds the threshold.

14. The surgical instrument of Claim 8, wherein the control circuit is configured to control the motor to decrease the speed at which the clamping member is driven.
